# EUROPEAN PATENT SPECIFICATION

(11) **EP 3 904 495 B1**
(45) Date of publication and mention of the grant of the patent: **04.02.2026**
(21) Application number: 19902133.8
(22) Date of filing: 13.12.2019
(51) Int. Cl.: G16H 50/20, G01N 33/49, G01N 33/50, G01N 33/569, G01N 33/68

(54) **BLOOD ANALYSIS DEVICE, COMPUTER PROGRAM, AND BLOOD ANALYSIS METHOD**
BLUTANALYSEVORRICHTUNG, COMPUTERPROGRAMM UND BLUTANALYSEVERFAHREN
DISPOSITIF D'ANALYSE DE SANG, PROGRAMME INFORMATIQUE ET PROCÉDÉ D'ANALYSE DE SANG

(30) Priority: 27.12.2018 JP 2018246186
(43) Date of publication of application: 03.11.2021
(73) Proprietor: HORIBA, Ltd., Kyoto 601-8510 (JP)
(72) Inventor: NISHIMORI, Masashi, Kyoto 601-8510 (JP); NISHIMURA TANIKAWA Jun Ivan, Kyoto 601-8510 (JP); OHASHI, Akika, Kyoto 601-8510 (JP)
(74) Representative: Isarpatent
(86) International application number: PCT/JP2019/049054
(87) International publication number: WO 2020/137640

(56) References cited:
- WO-A1-2012/016333
- JP-A- 2007 525 674
- JP-A- 2018 072 337
- JP-A- 2018 200 322
- US-A1- 2005 221 396
- LUÍS A. B. CRUZ, BARRAL-NETTO MANOEL, ANDRADE BRUNO B.: "Distinct inflammatory profile underlies pathological increases in creatinine levels associated with Plasmodium vivax malaria clinical severity", PLOS NEGLECTED TROPICAL DISEASES, vol. 12, no. 3/e0006306, 29 March 2018 (2018-03-29), pages 1 - 14, XP055723170
- FILIP C. CASTBERG, SARBAH EDEM W., KORAM KWADWO A., OPOKU NICHOLAS, OFORI MICHAEL F., STYRISHAVE BJARNE, HVIID LARS, KURTZHALS JØR: "Malaria causes long-term effects on markers of iron status in children: a critical assessment of existing clinical and epidemiological tools", MALARIA JOURNAL, vol. 17, no. 464, 11 December 2018 (2018-12-11), pages 1 - 12, XP055723168

## Description

### [Technical Field]

The present invention relates to a blood analysis device provided with the function of determining whether a blood specimen has a likelihood of being malaria positive, and a computer program and a method for determining whether a blood specimen has a likelihood of being malaria positive.

### [Background Art]

In the present specification, "malaria positive" refers to the presence of malaria plasmodium in the blood, and "malaria negative" refers to the absence of malaria plasmodium in the blood. In the following, the determination of whether a blood specimen has a likelihood of being malaria positive is to be also referred to as "malaria determination".

Conventionally, blood analysis devices provided with the function of performing malaria determination of blood specimens are known (patent documents 1, 2, and the like). In the blood analysis devices described in patent documents 1 and 2, malaria plasmodium present in malaria positive blood is regarded as a particle having a specific volume, and volume frequency distribution is analyzed with respect to particles of what volume are present at what frequency in a sample liquid prepared from the blood specimen of patients, and malaria determination is automatically performed based on the volume frequency distribution.

### [Document List]

### [Patent documents]

patent document 1: JP 2007-525674 A
patent document 2: JP 2007-024844 A
patent document 3: JP H11-101798 A
US 2005/221396 A1 relates to a method for detection of malaria and other parasite infections.
WO 2012/016333 A relates to a biomarker method for malaria. Luis A. B. Cruz, Barral-Netto Manoel, Andrade Bruno B., "Distinct inflammatory profile underlies pathological increases in creatinine levels associated with Plasmodium vivax malaria clinical severity", PLOS Neglected Tropical Diseases, (20180329), vol. 12, no. 3/e0006306, pages 1 - 14 and Filip C. Castberg, Sarbah Edem W., Koram Kwadwo A., Opoku Nicholas, Ofori Michael F., Styrishave Bjarne, Hviid Lars, Kurtzhals Jørgen A. L., "Malaria causes long-term effects on markers of iron status in children: a critical assessment of existing clinical and epidemiological tools", Malaria Journal, (20181211), vol. 17, no. 464, pages 1 - 12, both relates to malaria detection methods using biomarkers.

### [Summary of Invention]

### [Technical Problem]

However, the present inventors studied in detail the aforementioned conventional blood analysis devices provided with the function of performing malaria determination, and found that such blood analysis devices essentially require a comparatively highly functional and expensive constitution in order to perform malaria determination, and thus are not suitable for wide use in areas where the malaria determination function is necessary on a daily basis. Therefore, it was found that a determination method according to which malaria determination can be performed using a device with an inexpensive constitution and less functions is demanded.

The purpose of the present invention is to solve the aforementioned problems, provide a blood analysis device capable of performing malaria determination even though the constitution is inexpensive, and provide a computer program for the blood analysis device.

### [Solution to Problem]

The solution of the invention is defined in the independent claims 1, 9 and 14.

### SUMMARY OF THE DISCLOSURE

The disclosure provides example embodiments as follows.
[1] A blood analysis device comprising a measured value receiving part and a determining part, wherein
   the measured value receiving part receives measured test values of a blood specimen to be analyzed,
   the aforementioned measured test values are measured values of respective predetermined blood test items including a CRP value based on a CRP parameter value and a counted value of blood cells,
   the aforementioned measured value receiving part has a CRP value receiving part for receiving a CRP parameter value in a sample liquid comprising the aforementioned blood specimen, and
   the determining part determines whether the aforementioned blood specimen has a likelihood of being malaria positive based on a measured test value of a blood test item other than the CRP value and/or a specific measured test value regarding the CRP value.
[2] The blood analysis device of the aforementioned [1], wherein the aforementioned measured value receiving part has a volume value receiving part that receives a volume measured value of a particle in the sample liquid comprising the aforementioned blood specimen, and
   the aforementioned determining part determines that the aforementioned blood specimen has a high likelihood of being malaria positive when a frequency peak part exists in a predetermined volume range in a volume frequency distribution based on the aforementioned volume measured value, or when the CRP value based on the aforementioned CRP parameter value is not less than a threshold value determined in advance.
[3] The blood analysis device of the aforementioned [2], further comprising a leukocyte measurement chamber as a first particle measurement part, wherein
   in the leukocyte measurement chamber, a blood specimen to be supplied is subjected to a hemolysis treatment to lyse erythrocytes and diluted to form the aforementioned sample liquid, and a volume of each of leukocytes and particles other than leukocytes in the sample liquid is measured, and
   the volume value receiving part receives the aforementioned volume measured value from the aforementioned first particle measurement part.
[4] The blood analysis device of the aforementioned [2] or [3], further comprising a CRP measurement part that measures the aforementioned CRP parameter value, wherein
   the aforementioned CRP value receiving part receives the aforementioned CRP parameter value from the aforementioned CRP measurement part.
[5] The blood analysis device of any of the aforementioned [2] to [4], wherein the aforementioned determining part determines that the aforementioned blood specimen has a high likelihood of being malaria positive when the frequency peak part exists in the predetermined volume range in the volume frequency distribution based on the aforementioned volume measured value, and when the CRP value based on the aforementioned CRP parameter value is not less than the threshold value determined in advance.
[6] The blood analysis device of the aforementioned [1], wherein the aforementioned determining part is configured to use an important test item related to malaria positivity which is specified from predetermined blood test items by the following first learned model (M1), and determine whether the aforementioned blood specimen has a likelihood of being malaria positive based on a measured test value of the aforementioned important test item,
   wherein the aforementioned first learned model (M1) is formed by machine learning which of the aforementioned predetermined blood test items is related to malaria positivity by using, as teacher data, measured test value (A1) obtained for the aforementioned predetermined blood test item by using blood specimen (A) known to be malaria positive as a measuring object, and information (A2) that the measured test value (A1) is of a malaria positive blood specimen, and measured test value (B1) obtained for the aforementioned predetermined blood test item by using blood specimen (B) known to be malaria negative as a measuring object, and information (B2) that the measured test value (B1) is of a malaria negative blood specimen.
[7] The blood analysis device of the aforementioned [6], wherein the aforementioned important test item includes one or more selected from the group consisting of
   whether a frequency peak exists in a volume range of 25fL to 45fL in a volume frequency distribution of leukocytes in a blood specimen to be analyzed,
   leukocyte count of a blood specimen to be analyzed,
   platelet count of a blood specimen to be analyzed, and
   CRP value of a blood specimen to be analyzed.
[8] The blood analysis device of the aforementioned [1], wherein the aforementioned determining part is configured to use a degree of relevance to each of predetermined blood test items for malaria positivity which is specified by the following second learned model (M2), and determine whether the aforementioned blood specimen has a likelihood of being malaria positive based on a measured test value of the aforementioned predetermined blood test item and the aforementioned specified degree of relevance,
   wherein the aforementioned second learned model (M2) is formed by machine learning which of the aforementioned predetermined blood test items is related to malaria positivity in what degree by using, as teacher data, measured test value (A1) obtained for the aforementioned predetermined blood test item by using blood specimen (A) known to be malaria positive as a measuring object, and information (A2) that the measured test value (A1) is of a malaria positive blood specimen, and measured test value (B1) obtained for the aforementioned predetermined blood test item by using blood specimen (B) known to be malaria negative as a measuring object, and information (B2) that the measured test value (B1) is of a malaria negative blood specimen.
[9] A computer program that allows a computer to perform a step of receiving measured test values of a blood specimen to be analyzed, wherein the aforementioned measured test values are measured values of respective predetermined blood test items including a CRP value and a counted value of blood cells, and
   a step of determining whether the aforementioned blood specimen has a likelihood of being malaria positive based on a measured test value for a specific blood test item other than the CRP value and/or a measured test value regarding the CRP value.
[10] The computer program of the aforementioned [9], wherein the computer performs a step of receiving a volume measured value of a particle in the sample liquid comprising the aforementioned blood specimen,
   a step of receiving a CRP parameter value in the aforementioned sample liquid,
   a step of determining whether a frequency peak part exists within a volume range unique to malaria plasmodium in the aforementioned volume frequency distribution based on the aforementioned volume measured value,
   a step of determining whether the aforementioned CRP value based on the CRP parameter value is not less than a threshold value determined in advance, and
   a step of determining that the aforementioned blood specimen has a high likelihood of being malaria positive when the aforementioned frequency peak part exists or when the aforementioned CRP value is not less than the aforementioned threshold value.
[11] The computer program of the aforementioned [10], further comprising a computer program that allows a computer to perform a step of determining that the aforementioned blood specimen has a high likelihood of being malaria positive when the aforementioned frequency peak part exists and the aforementioned CRP value is not less than the aforementioned threshold value.
[12] The computer program of the aforementioned [9], wherein the aforementioned determination step comprises a step of using an important test item related to malaria positivity which is specified from predetermined blood test items by the following first learned model (M1), and determining whether the aforementioned blood specimen has a likelihood of being malaria positive based on a measured test value of the aforementioned important test item,
   wherein the aforementioned first learned model (M1) is formed by machine learning which of the aforementioned predetermined blood test items is related to malaria positivity by using, as teacher data, measured test value (A1) obtained for the aforementioned predetermined blood test item by using blood specimen (A) known to be malaria positive as a measuring object, and information (A2) that the measured test value (A1) is of a malaria positive blood specimen, and measured test value (B1) obtained for the aforementioned predetermined blood test item by using blood specimen (B) known to be malaria negative as a measuring object, and information (B2) that the measured test value (B1) is of a malaria negative blood specimen.
[13] The computer program of the aforementioned [9], wherein the aforementioned determination step comprises a step of using a degree of relevance to each of predetermined blood test items for malaria positivity which is specified by the following second learned model (M2), and determining whether the aforementioned blood specimen has a likelihood of being malaria positive based on a measured test value of the aforementioned predetermined blood test item and the aforementioned specified degree of relevance,
   wherein the aforementioned second learned model (M2) is formed by machine learning which of the aforementioned predetermined blood test items is related to malaria positivity in what degree by using, as teacher data, measured test value (A1) obtained for the aforementioned predetermined blood test item by using blood specimen (A) known to be malaria positive as a measuring object, and information (A2) that the measured test value (A1) is of a malaria positive blood specimen, and measured test value (B1) obtained for the aforementioned predetermined blood test item by using blood specimen (B) known to be malaria negative as a measuring object, and information (B2) that the measured test value (B1) is of a malaria negative blood specimen.
[14] A method for analyzing blood, comprising a step of preparing measured test values of a blood specimen to be analyzed, wherein the aforementioned measured test values are measured values of respective predetermined blood test items including a CRP value and a counted value of blood cells, and
   a step of determining whether the aforementioned blood specimen has a likelihood of being malaria positive based on a measured test value for a specific blood test item other than the CRP value and/or a measured test value regarding the CRP value.
[15] The blood analysis method of the aforementioned [14], wherein the aforementioned determination step comprises a step of using an important test item related to malaria positivity which is specified from predetermined blood test items by the following first learned model (M1), and determining whether the aforementioned blood specimen has a likelihood of being malaria positive based on a measured test value of the aforementioned important test item,
   wherein the aforementioned first learned model (M1) is formed by machine learning which of the aforementioned predetermined blood test items is related to malaria positivity by using, as teacher data, measured test value (A1) obtained for the aforementioned predetermined blood test item by using blood specimen (A) known to be malaria positive as a measuring object, and information (A2) that the measured test value (A1) is of a malaria positive blood specimen, and measured test value (B1) obtained for the aforementioned predetermined blood test item by using blood specimen (B) known to be malaria negative as a measuring object, and information (B2) that the measured test value (B1) is of a malaria negative blood specimen.
[16] The blood analysis method of the aforementioned [14], wherein the aforementioned determination step comprises a step of using a degree of relevance to each of predetermined blood test items for malaria positivity which is specified by the following second learned model (M2), and determining whether the aforementioned blood specimen has a likelihood of being malaria positive based on a measured test value of the aforementioned predetermined blood test item and the aforementioned specified degree of relevance,
   wherein the aforementioned second learned model (M2) is formed by machine learning which of the aforementioned predetermined blood test items is related to malaria positivity in what degree by using, as teacher data, measured test value (A1) obtained for the aforementioned predetermined blood test item by using blood specimen (A) known to be malaria positive as a measuring object, and information (A2) that the measured test value (A1) is of a malaria positive blood specimen, and measured test value (B1) obtained by measuring for the aforementioned predetermined blood test item by using blood specimen (B) known to be malaria negative as a measuring object, and information (B2) that the measured test value (B1) is of a malaria negative blood specimen.

### [Advantageous Effects of Invention]

The present invention makes it possible to perform malaria determination even though the device constitution is inexpensive.

Conventionally, since the effectiveness of the complementary use of CRP value and volume frequency distribution in malaria determination has not been found, the volume frequency distribution of particles and the CRP value were not correlated with each other as conditions for malaria determination. In the present invention, it has been found that the accuracy or reliability of malaria determination is synergistically improved when malaria determination is performed based on both the volume frequency distribution of particles and the CRP value.

In a preferred embodiment of the present invention, an artificial intelligence undergoes machine learning of [correlation between respective measured test values relating to predetermined blood test items of blood specimen known to be malaria positive, and the fact of being malaria positive] and [correlation between respective measured test values relating to predetermined blood test items of blood specimen known to be malaria negative, and the fact of being malaria negative] to construct a learned model of correlation between predetermined blood test items and malaria positivity (which blood test item from among predetermined blood test items is related to malaria positivity, or the level of relationship with malaria positivity). Using the learned model, specific blood test items (important test items) related to malaria positivity are selected from the predetermined blood test items (i.e., various blood test items), and the specific measured test values are used for malaria determination. As a result, new blood test items can be added for determination of malaria positivity in addition to the blood test items conventionally used by medical workers such as doctors, engineers and the like for malaria determination as being empirically important, which in turn improves the accuracy of malaria determination.

### [Brief Description of Drawings]

Fig. 1 is a block diagram showing one embodiment of a preferred constitution of the blood analysis device of the present invention.
Fig. 2 is a flowchart showing the action of the blood analysis device of the present invention. The flowchart also explains the constitution of the computer program of the present invention.
Fig. 3 is a graph showing an example of a frequency peak part that appears due to the presence of malaria plasmodium in the volume frequency distribution chart of particles in a sample liquid (blood specimen after hemolysis treatment and diluting). The Figure shows frequency distribution in a region where the volume of particles is small.
Fig. 4 is a scatter diagram showing the relationship between the height of a frequency peak part that appears due to the presence of malaria plasmodium in the volume frequency distribution chart of particles in a sample liquid, and the CRP value of the sample liquid.
Fig. 5 is a graph showing that the height of a frequency peak part that appears due to the presence of malaria plasmodium in the volume frequency distribution chart of particles in a sample liquid changes according to the kind of the malaria plasmodium.
Fig. 6 is a graph showing an example of a frequency peak part that appears due to infection with dengue virus in the volume frequency distribution chart of particles in a sample liquid drawn from a dengue virus-positive blood specimen. The Figure shows frequency distribution in a region where the volume of particles is small, as in Fig. 3.
Fig. 7 is a box plot showing the CRP value of each of a malaria-positive blood specimen and a dengue fever-positive blood specimen. Fig. 7(a) is a graph relating to the malaria-positive blood specimen and Fig. 7(b) is a graph relating to the dengue fever-positive blood specimen.
Fig. 8 is a graph showing a correlation between [height of frequency peak appearing in 25fL - 45fL volume range in a graph of volume frequency distribution obtained by counting leukocytes in malaria-positive blood] and [number of malaria plasmodiums contained per unit volume of the blood].
Fig. 9 is a flowchart showing the steps of the blood analysis method of the present invention. The flowchart also explains a preferred embodiment of the computer program of the present invention.

### [Description of Embodiments]

### [constitution example of the blood analysis device of the present invention]

The blood analysis device of the present invention (hereinafter to be also referred to as the device) is described in detail by illustrating examples.

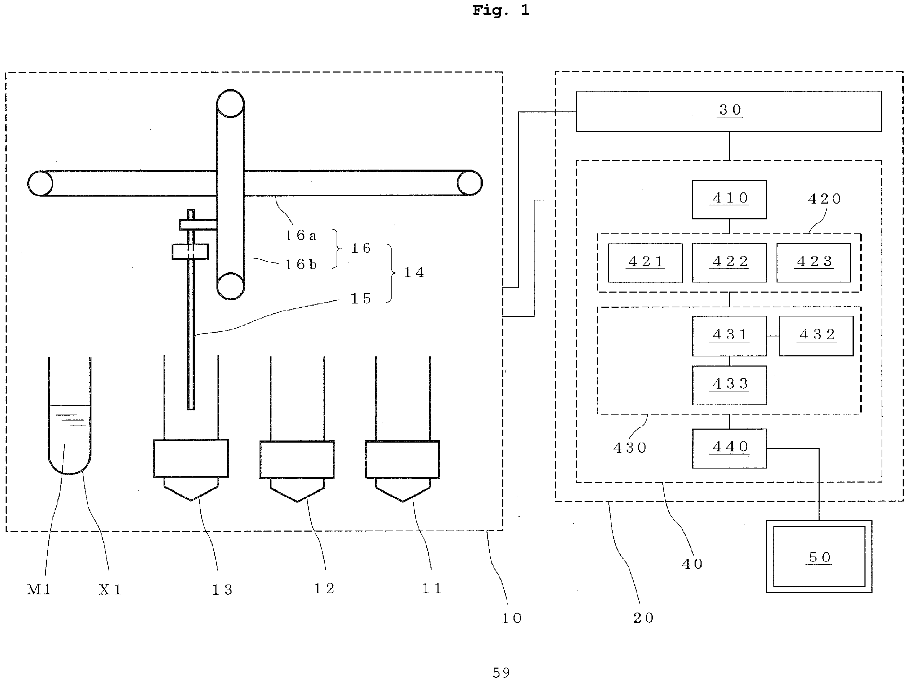

One embodiment of the constitution is shown in Fig. 1, in which the device is configured to have at least a measured value receiving part 410 and a determining part 430. The measured value receiving part 410 is a part configured to receive predetermined measured test values of a blood specimen to be analyzed, and includes a CRP value receiving part. In the example of Fig. 1, a measured value receiving part 410 has a volume value receiving part and a CRP value receiving part.

The aforementioned measured test values are measured values of respective predetermined blood test items (conventionally-known various blood test items). In a preferred embodiment of the present invention, a CRP value and a counted value of blood cells are included in the blood test items; however, the CRP value may not be included in some cases. The measured test values may be values of electric signals as output from a blood measurement device, or values obtained by arithmetical processing, or values converted into those having a unit that can be understood by medical workers.

The determining part 430 is a part where malaria determination of a blood specimen to be analyzed is performed. The determining part is a part configured to perform the malaria determination based on one or both of the [measured test values of blood test items other than CRP value] and the [measured test values of CRP value].

In the example shown in Fig. 1, the device is configured to receive a specimen container X1 containing a blood specimen M1 at a predetermined position, and measure and analyze a blood specimen M1 in the specimen container X1. The device has a measuring mechanism part 10 (including the first particle measurement part 11 and a CRP measurement part 13) that performs particle measurement and CRP value measurement, and a control part 20 (including analysis part 40) that controls the measuring mechanism part 10 and analyzes the measured values. In the example of Fig. 1, the first particle measurement part 11 is a leukocyte measurement chamber (hereinafter WBC chamber to be also referred to as), and the CRP measurement part 13 is a CRP measurement chamber (hereinafter to be also referred to as CRP chamber). In the example of Fig. 1, the second particle measurement part 12 is further provided as a preferred embodiment, and the second particle measurement part 12 is an erythrocyte measurement chamber (to be also referred to as RBC chamber). A detailed explanation of the device as a whole is given later. In the constitution shown in Fig. 1, it is important that the device has at least the below-mentioned volume value receiving part, a CRP value receiving part, and a determining part in the analysis part.

The first particle measurement part (WBC chamber) 11 is configured to measure the volume of particles (particles containing leukocyte) in a sample liquid as a measurement target, and output the volume measured value of each particle. The sample liquid to be measured in the first particle measurement part 11 contains a blood specimen M1 that underwent a hemolysis treatment. In a preferred embodiment, the blood specimen M1 is subjected to a hemolysis treatment and diluted. In the first particle measurement part 11 shown in Fig. 1, a measurement container (chamber) is provided with a particle counting mechanism for measuring the volume of each particle.

A CRP measurement part (CRP chamber) 13 is configured to measure a CRP parameter value which is an indicated value corresponding to the CRP value of the blood specimen M1, and output the measured value. In the CRP measurement part 13 shown in Fig. 1, a measurement container (chamber) is provided with an optical device for measuring the CRP parameter value.

The analysis part 40 has at least a volume value receiving part, a CRP value receiving part, and a determining part 430. In the example shown in Fig. 1, a volume value receiving part and a CRP value receiving part are contained in a measured value receiving part 410. In the example shown in Fig. 1, the analysis part 40 has a frequency distribution calculating part 421, and a CRP value determining part 423. The frequency distribution calculating part 421 receives a volume measured value of each particle from the first particle measurement part 11, and calculates the volume frequency distribution of particles (particles containing leukocyte) in the blood specimen M1. The particles contain malaria plasmodium in a malaria-positive blood specimen. The CRP value determining part 423 receives the CRP parameter value output from the CRP measurement part 13, and determines the CRP value of the blood specimen M1. For this end, in the example of Fig. 1, a measured value receiving part (including volume value receiving part and CRP value receiving part) 410and a measured value processing part 420 are provided in the analysis part 40.

The measured value receiving part (including volume value receiving part and CRP value receiving part) 410 receives measured values output from each measurement part (11, 12, 13) in the measuring mechanism part 10 as arithmetically processable data. The measured value receiving part 410 may be configured to contain an interface as a hardware, and a software (computer program) that holds the received measured value signals as a data set of the measured values. The measured value receiving part 410 may store the received respective measured values in a storage device (not shown).

The measured value processing part 420 contains the first frequency distribution calculating part 421 and a CRP value determining part 423. In the example of Fig. 1, the measured value processing part 420 also contains the second frequency distribution calculating part 422.

The first frequency distribution calculating part 421 processes the measured values output from the WBC chamber 11 and calculates the volume frequency distribution of particles (leukocyte, malaria plasmodium) in the hemolysis-treated blood specimen M1.

The CRP value determining part 423 processes the measured values (CRP parameter) output from the CRP chamber 13 and determines of the CRP value.

The second frequency distribution calculating part 422 processes the measured values output from the second particle measurement part (RBC chamber) and calculates the volume frequency distribution of particles (particles containing erythrocyte, platelet) in the blood specimen M1.

The determining part 430 determines that blood specimen M1 has a high likelihood of being malaria positive when the blood analysis value obtained by the measured value processing part 420 (particularly, the volume frequency distribution obtained by the first frequency distribution calculating part 421, and the CRP value obtained by the CRP value determining part 423) satisfies one of the following conditions (i) and (ii) satisfied. In a preferred embodiment, blood specimen M1 is determined to have a high likelihood of being malaria positive (or blood specimen M1 is determined to have a higher likelihood of being malaria positive) when both the following conditions (i) and (ii) are satisfied.
(i) A frequency peak part exists within a volume range unique to malaria plasmodium in the aforementioned volume frequency distribution.
(ii) The CRP value is not less than the threshold value determined in advance.

While the detail is described later, in the example of Fig. 1, the determining part 430 is provided with a condition determining part 431, a reference data holding part 432, and a determining part 433 in order to perform determination of the aforementioned conditions (i) and (ii). The reference data holding part 432 stores the data to be referred to for the determination by the condition determining part 431, such as threshold value for determination of the CRP value and the like.

The condition determining part 431 refers to necessary data held by the reference data holding part 432 and performs determination of the aforementioned conditions (i) and (ii).

The determining part 433 receives the results of the condition determination by the condition determining part 431, and selects the determination of "high likelihood of malaria positivity" when only one of the aforementioned conditions (i) and (ii) is satisfied. When neither of the aforementioned conditions (i) and (ii) is satisfied, the determination of "not malaria positive" may be selected. Even when both of the aforementioned conditions (i) and (ii) are satisfied, the determination of "high likelihood of malaria positivity" may be selected. When both of the aforementioned conditions (i) and (ii) are satisfied, the likelihood of malaria positivity is higher (or accuracy of the determination of high likelihood of malaria positivity is higher) than when only one of them is satisfied. Therefore, when both of the aforementioned conditions (i) and (ii) are satisfied, the determination of "higher likelihood of malaria positivity", alternatively, "malaria positive" may be selected to make distinction on determination from the case where only one of them is satisfied. In the example of Fig. 1, the analysis part 40 has a determination result output part 440, through which the determination results selected by the determining part 433 is output on a peripheral equipment such as display device 50 and the like, communication equipment, external computer or the like.

As other embodiment of the analysis part, the measured value receiving part 410 may receive an already-calculated frequency distribution. In addition, the CRP parameter value may be a CRP value itself.

With the above configuration, the device can perform malaria determination even though it has an inexpensive configuration. The device may be an analysis device provided only with a first particle measurement part, a CRP measurement part, and a control part (including an analysis part), or may be an analysis device exclusively for malaria determination that outputs only the result of malaria determination. However, it is preferably a device that has a configuration of a conventionally-known blood analysis device added with an analysis function for malaria determination, as shown in Fig. 1, and performs analysis of complete blood count, analysis of CRP value, and malaria determination. The example shown in Fig. 1 is a blood analysis device provided with a WBC chamber 11, an RBC chamber 12, and a CRP chamber 13, in which the WBC chamber 11 is utilized as the first particle measurement part. This is because, in the WBC chamber, a blood specimen undergoes a hemolysis treatment (treatment to dissolve erythrocyte with hemolytic agent), is diluted to form a sample liquid, and leukocytes are counted. Thus, the WBC chamber is suitable for exposing malaria plasmodium lurking in erythrocytes and counting them as particles. It is also preferable to utilize the measurement results of platelets counted in the RBC chamber 12 in order to further improve the accuracy and reliability of malaria determination (described later).

### [particle counting mechanism]

Particle counting includes not only simply counting the number of particles, but also measuring the volume of each particle. This makes it possible to analyze particles of what volume are present in what number (that is, volume frequency distribution of particles). The "frequency" in the volume frequency distribution is the number of particles per each volume (or channel described later).

Examples of the particle counting mechanism in the particle measurement part include a mechanism of performing an impedance method (electrical resistance method) (mechanism of measuring volume of particles by using a change in the electric property (impedance) in aperture when the particles pass through the aperture), a mechanism of performing flow cytometry (irradiating predetermined irradiation light to particles in a sample liquid advancing through a flow path in a flow cell, obtaining optical properties such as light scattering, light absorbance and the like, and measuring the volume of the particles from the optical properties), and a light-focused flow impedance method (mechanism for performing an impedance method and flow cytometry in one flow path so that each particle can be measured by the impedance method and flow cytometry). Among these particle counting mechanisms, the mechanism for performing the impedance method is inexpensive and is a useful mechanism for finding a frequency peak part unique to malaria plasmodium in the frequency distribution.

### [particle measurement part mounted on the device]

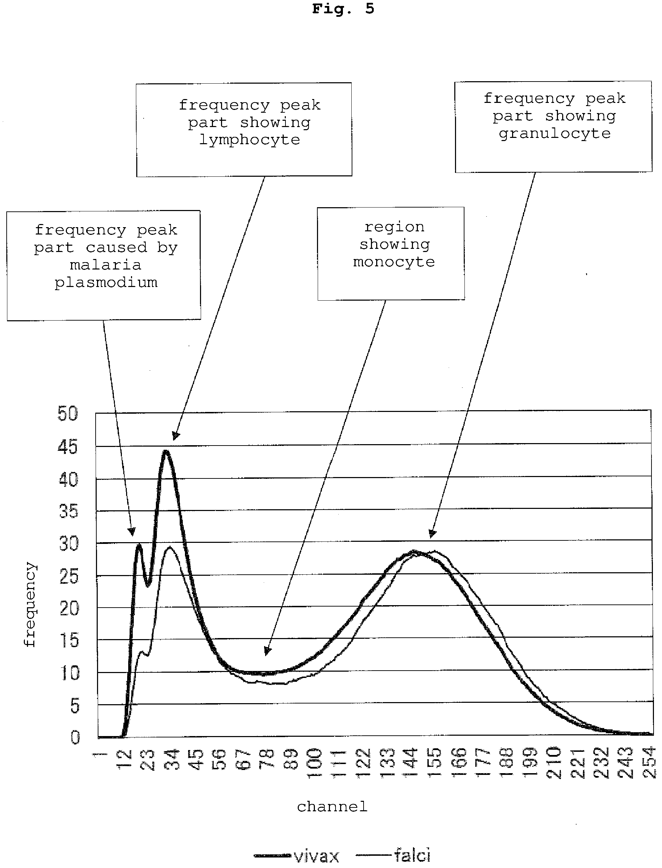

In the example of Fig. 1, the first particle measurement part (WBC chamber) is configured to count particles by the below-mentioned impedance method. The analysis part is configured to classify leukocytes into lymphocyte, monocyte, and granulocyte (classification of leukocyte into 3 types), and display the state of the frequency distribution thereof as analysis results, as shown in the volume frequency distribution in Fig. 5. In the RBC chamber, platelets may also be counted, and the WBC chamber may be configured to measure the hemoglobin concentration (Hgb). In addition, it may be configured to perform measurement of items similar to those in conventional blood analysis devices such as erythrocyte volume (MCV), hematocrit value (Hct) and the like.

In addition, a particle measurement part (measurement chamber) for classifying granulocytes into neutrophil, eosinophil, and basophil may be added to the device. Examples of the particle measurement part include LMNE chamber (measurement chamber for counting lymphocytes, monocytes, neutrophils, eosinophils by using cell staining technique and particle counting mechanism (particularly, mechanism for light-focused flow impedance method)), and BASO chamber (measurement chamber for hemolysis and contraction of components other than basophil by hemolytic agent, and counting basophils by particle counting mechanism).

A blood analysis device provided with two measurement chambers (RBC chamber and WBC chamber for classification of leukocytes into 3 types) configured to perform an impedance method, and a CRP chamber can perform basic analyses of blood with an inexpensive constitution. Therefore, a preferable blood analysis device useful for malaria determination and medical diagnosis can be provided at a low cost.

### [CRP measurement part mounted on the device]

The CRP chamber is configured to measure a CRP parameter value corresponding to the CRP value of a blood specimen. Examples of the method for obtaining a CRP parameter value in a measurement chamber include CRP latex immunity nephelometry RATE method and the like. For example, in the CRP latex immunity nephelometry RATE method, a latex reagent for immunoassay (e.g., anti-human CRP sensitized latex immunity reagent and the like) is added to a blood specimen in the chamber. A photoirradiation part and a photodetection part are provided on the lower wall surface of the chamber, and the intensity of the detected transmitted light is output as a CRP parameter value. This CRP parameter value is associated with the CRP value in the CRP value determining part 423 of the analysis part 40. In another embodiment, the CRP measurement part has a CRP value determining part which may output the CRP parameter value as a data converted to the CRP value.

For the technique itself for measuring the CRP value in the blood analysis device, conventionally-known techniques such as the aforementioned patent document 3 and the like can be referred to.

### [measuring mechanism part]

Each part of the measuring mechanism part 10 is controlled and operated by a mechanism control part 30 contained in the control part 20. The mechanism control part 30 and the analysis part 40 cooperate with each other to allow the mechanism control part 30 to perform measurement, blood analysis, and malaria determination.

In the example of Fig. 1, the measuring mechanism part (broken line indicate) 10 has the CRP chamber 13, the RBC chamber 12, the WBC chamber 11, and a dispensing mechanism 14. The dispensing mechanism 14 has a sampling nozzle 15 and a transfer mechanism 16, and the transfer mechanism is configured to contain a horizontal transfer mechanism 16a and a vertical transfer mechanism 16b so that the sampling nozzle 15 can be moved in the horizontal direction and the vertical direction. The sampling nozzle 15 is an elongated tube under the control of the mechanism control part 30 and moves to the specimen container 10, a reagent container, the erythrocyte measurement chamber, and the leukocyte measurement chamber to suck and discharge a blood specimen, a reagent liquid, a sample liquid produced in a chamber, and the like.

In addition to these, the measuring mechanism part 10 is appropriately provided with reagent containers (plural) storing reagents necessary for the measurement, a dilution mechanism, a pump for sucking/discharging, a syringe, an electromagnetic valve, and the like (not shown). For the measuring mechanism part, conventionally-known particle analysis devices, blood analysis devices, and blood cell count devices as in the aforementioned patent document 3 and the like can be referred to.

### [control part]

The control part may be constructed with a logic circuit and the like, but a computer is suitable. In the computer, a computer program configured to control action of each part of the measuring mechanism part and perform arithmetic operations for analysis is executed.

### [main operation and analysis flow of the device]

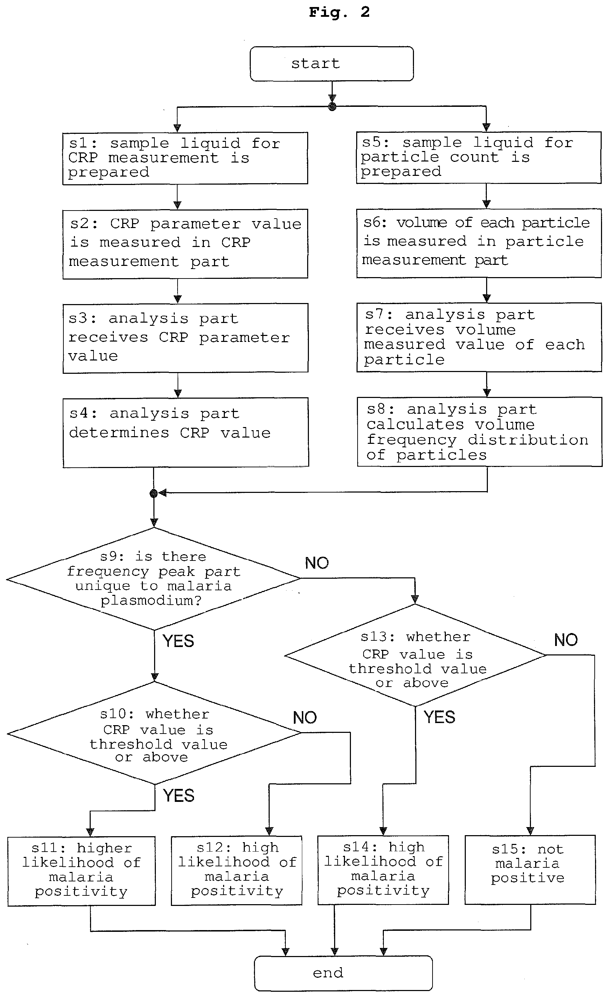

Fig. 2 is a flowchart illustrating an outline of the operation of the device. In the example of the flowchart, while analysis steps s1 - s4 of the CRP value and analysis steps s5 - s8 of the particle measurement (leukocyte count) are shown in parallel, the operations of these two series may be serial. The number assigned to each step is for identification. In practice, an RBC chamber and an additional measurement chamber are added, blood specimens are distributed to each chamber in a predetermined order to form sample liquids there (also including the transfer of sample liquids between chambers; for example, a portion of blood specimen diluted in WBC chamber is transferred to RBC chamber where it is further diluted), after which measurement proceeds independently in each chamber and the time required for the measurement is also independent for each chamber. Hereinafter one example of the main operation and analysis flow in the device of Fig. 1 is described below.

### [CRP value analysis steps s1 - s4]

### (step s1)

The sampling nozzle (hereinafter nozzle) acts to measure the CRP parameter value, supplies predetermined amounts of the reagent necessary for measuring the CRP parameter value (reagent container not shown) and blood specimen M1 in the specimen container X1 into the CRP measurement part (CRP chamber) 13, where a sample liquid for measuring the CRP parameter value is formed. The nozzle is washed as appropriate.

### (step s2)

CRP parameter value is measured in the CRP chamber, and the measured value is output to the analysis part.

### (step s3)

The analysis part 40 (the measured value receiving part 410) receives the CRP parameter value (absorbance).

### (step s4)

The analysis part 40 (the CRP value determining part 423 of the measured value processing part 420) determines CRP value from CRP parameter value. The CRP value determining part 423 may have a reference table for converting CRP parameter value to CRP value.

### [particle volume frequency distribution analysis steps s5 - s8] (step s5)

The nozzle supplies a predetermined amount of blood specimen M1 in the specimen container X1 to the first particle measurement part (WBC chamber) 11, and a predetermined amount of a diluent (water, saline, phosphate buffer diluent or the like) is supplied thereto to dilute blood specimen M1. While the flowchart of Fig. 2 does not specifically show the detailed action, a part of the blood specimen diluted in the WBC chamber is transferred to the RBC chamber, further diluted therein, and erythrocytes and platelets are counted. For the dilution rates of these, conventionally-known counting techniques can be referred to. In the WBC chamber, a hemolytic agent that further dissolves erythrocytes (hemolysis) is added to the diluted blood specimen to form a sample liquid in which erythrocytes are dissolved.

### (step s6)

In the first particle measurement part (WBC chamber) 11, particles are counted, the volume of each particle is measured, and the measured values are output to the analysis part. In the WBC chamber, a hemoglobin concentration is measured by an optical device for a colorimetric method (non-cyan method), and the measured values may be output. On the other hand, in the second particle measurement part (RBC chamber) 12, the particles are also counted, the volume of each particle is measured, and the measured values are output to the analysis part.

### (step s7)

The analysis part 40 (the measured value receiving part 410) receives the measurement results such as particle volume measured value and the like from each particle measurement part.

### (step s8)

The analysis part 40 (the frequency distribution calculating part 421 of the measured value processing part 420) processes particle volume measured value from the WBC chamber, and calculates volume frequency distribution of particles containing leukocytes. On the other hand the second frequency distribution calculating part 422 processes particle volume measured value from the RBC chamber, and calculates volume frequency distributions of erythrocytes and platelets. These volume frequency distributions may be output or displayed as histograms.

### [analysis steps s9 - s15 of malaria determination in determining part 430]

The condition determination shown in the flowchart of Fig. 2 is an example in which the determination of the aforementioned condition (i) is performed first, and given the results thereof, the determination of the aforementioned condition (ii) is performed. The order of the condition determination relating to the volume frequency distribution and the condition determination relating to the CRP value may be reversed or concurrent.

### (step s9)

In the example of the flowchart of Fig. 2, the condition determining part 431 in the determining part 430 determines whether the volume frequency distribution obtained in the measurement by the WBC chamber in step s9 satisfies the aforementioned condition (i) (frequency peak part exists within a volume range unique to malaria plasmodium).

### (step s10)

In step s9, when the condition determining part 431 determines that a frequency peak part exists (YES), the condition determining part 431 determines whether the aforementioned condition (ii) (CRP value is not less than the threshold value determined in advance) is satisfied in step s10.

### (step s11)

In step s10, when the condition determining part 431 determines that the CRP value is not less than the threshold value (YES), since both the aforementioned condition (i) and the aforementioned condition (ii) are satisfied, the determining part 433 selects a determination result of "higher likelihood of malaria positivity" in step s11. This determination result shows preferable one embodiment of setting the levels in the likelihood of malaria positivity. In steps s11, s12, and s14, the determination result of "high likelihood of malaria positivity" may be similarly selected.

### (step s12)

In step s10, when the condition determining part 431 determines that the CRP value is less than the threshold value (NO), the determining part 433 selects the determination result of "high likelihood of malaria positivity" in step s12.

### (step s13)

On the other hand, in step s9, when the condition determining part 431 determines no frequency peak part (NO), the condition determining part 431 determines whether the aforementioned condition (ii) (CRP value is not less than the threshold value determined in advance) is satisfied in step s13.

### (step s14)

In step s13, when the CRP value is determined to be not less than the threshold value (YES), the determining part 433 selects the determination result of "high likelihood of malaria positivity" in step s14.

### (step s15)

In step s13, when the condition determining part 431 determines that the CRP value is less than the threshold value (NO), the determining part 433 selects the determination result of "not malaria positive" in step s15.

The malaria determination is preferably performed by the above steps.

### [detailed explanation on the aforementioned conditions (i), (ii)]

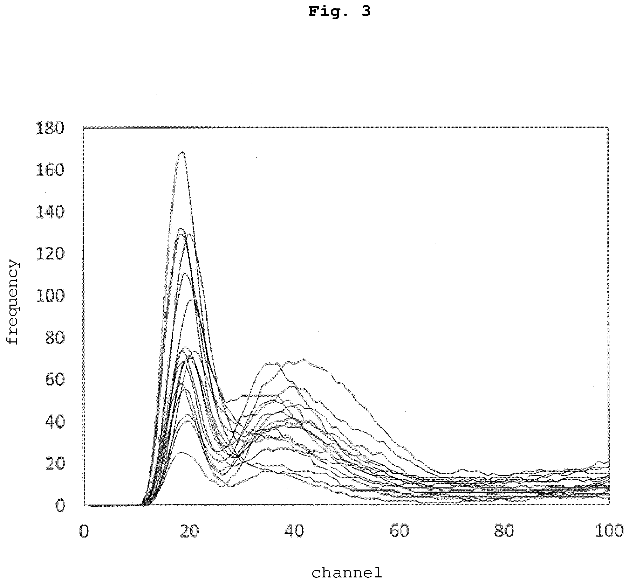

As shown in the below-mentioned Experimental Example 1 and the volume frequency distribution chart of Fig. 3, it was found in the present invention that a unique frequency peak part sometimes appears within a predetermined range in the volume frequency distribution chart of particles in a sample liquid (blood specimen after hemolysis treatment and diluting) due to the presence of malaria plasmodium. In the example of volume frequency distribution chart of Fig. 3, a clear frequency peak part appears within the range of channels 14 - 26 corresponding to 25fL (femtoliter) - 45fL, particularly, within the range of channels 17 - 23 corresponding to 30fL - 40fL, in a malaria-positive blood specimen (" channel " is described later). In the example of Fig. 3, the center of the frequency peak part is concentrated near channel 20. This is because malaria plasmodiums contained in erythrocytes became suspended in the sample liquid when the erythrocytes were dissolved, and counted as particles. The frequency peak part around channel 40 indicates the presence of lymphocytes, and the frequency peak part unique to malaria plasmodium is adjacent to the lymphocyte frequency peak part on the small volume side.

### [channel]

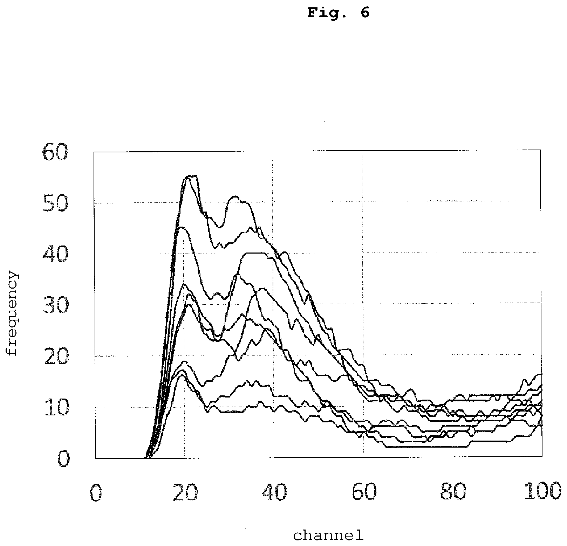

It is preferable to divide the overall width from the minimum value (may be zero) to the maximum value of the volume measured value showing the volume of particles into, for example, 256 steps (0 - 255), 1024 steps (0 - 1023) and the like rather than the specific numerical values. This facilitates handling of the data when the control part (computer) processes the data to determine the frequency distribution. Each section divided as mentioned above is also called a channel. In the present specification, examples using channel 0 to channel 255 as a name of the volume section are recited. Fig. 3 and Fig. 6 show frequency distribution of regions with small particle volume (channels 0 to 100) from among the total channels 0 to 255.

### [presence of frequency peak part unique to malaria plasmodium]

In the present invention, the presence of a frequency peak part in the volume frequency distribution of particles typically means a state in which the graph curve of the volume frequency distribution (a curve smoothly connecting the frequency of each channel) with a positive or negative slope of the tangent line contains a part with a 0 slope in the middle of the curve; in a more prominent state, a part with a maximum value exists. As shown in the graph curve of Fig. 3, the frequency of the channel on the side with a smaller volume than that of the frequency peak part is lower than the frequency of the frequency peak part, and therefore, the slope of the tangent line of the side with a smaller volume than that of the frequency peak part is a plus (positive).

The height of the frequency peak part unique to malaria plasmodium is preferably determined by a difference in height based on the frequency of the bottom of the valley part (peak to valley), the valley part being adjacent to the frequency peak part on the side with a larger volume. The height may also be based on zero frequency (= frequency value itself of frequency peak part), or based on a predetermined frequency, or the like.

### [first threshold value: lower limit of height of frequency peak part unique to malaria plasmodium]

According to the experiment conducted by the present inventors, for example, the height of the frequency peak part unique to malaria plasmodium (peak to valley) is one or more. That is, when the graph curve of the volume frequency distribution within the volume range unique to malaria plasmodium contains a part lacking a monotonous increase but showing a change in the direction of the curve like an inflection point (height of frequency peak part is zero), a likelihood of malaria positivity is suspected. In the present invention, 1, which is an appropriate value based on experiments, is adopted as the value of the lower limit in the determination of the height of the frequency peak part (peak to valley). The lower limit value "1" may be stored in advance in the reference data holding part 432. In addition, the lower limit value may be changed as appropriate by an operator of the device.

When a frequency peak part exists, the determining part determines high likelihood of malaria positivity, and a final malaria determination is made together with the determination result of the CRP value.

### [threshold value: lower limit of CRP value unique to malaria positivity]

The CRP value of healthy human is generally about 0 mg/L to 5 mg/L. According to the experiment conducted by the present inventors, a malaria-positive blood specimen sometimes shows a high CRP value of about 50 mg/L. From the experimental results, an appropriate threshold value permitting determination of high likelihood of malaria positivity (lower limit value for determination) is, for example, higher than 5 mg/L up to 50 mg/L, more preferably, 20 mg/L - 30 mg/L. The threshold value can be selected from these ranges. In the below-mentioned Experimental Example 2, a CRP value of 26 mg/L was adopted as the threshold value (lower limit value for determination), and high likelihood of malaria positivity was determined when the CRP value was not less than the threshold value. The adopted threshold value is stored in advance in the reference data holding part 432. The threshold value may be changed as appropriate by an operator of the device.

### [relationship between frequency peak part unique to malaria plasmodium and CRP value]

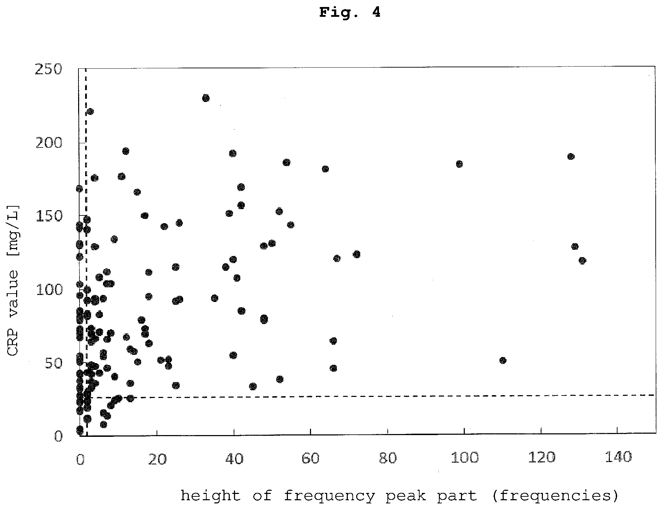

As shown in the below-mentioned Experimental Example 2 and the scatter diagram of Fig. 4, it has been found in the present invention that the accuracy or reliability of malaria determination is synergistically improved by malaria determination based on both the volume frequency distribution of particles and the CRP value.

According to the study by the present inventors, in the scatter diagram of Fig. 4, all cases where the height of the frequency peak part in the volume frequency distribution of particles containing leukocyte and malaria plasmodium is one or more are preferably determined to have high likelihood of malaria positivity. All cases where the CRP value is not less than 26 mg/L in the volume frequency distribution are preferably determined to have high likelihood of malaria positivity. In the scatter diagram of Fig. 4, when the height of the frequency peak part in the volume frequency distribution of particles containing leukocyte and malaria plasmodium is one or more, and the CRP value is not less than 26 mg/L, it is preferable to determine high (or higher) likelihood of malaria positivity.

### [indication of malaria determination results]

The results of malaria determination may be displayed using notation or symbol that ensures understanding of the operator of the blood analysis device or those who see the analysis results as to whether it is malaria positive, such as "(malaria) positive" and "high likelihood of (malaria) positivity".

In addition, a greater height of the frequency peak part (peak to valley) and/or a higher CRP value may indicate that the likelihood of malaria positivity is higher.

### [distinction of types of malaria plasmodium]

Malaria plasmodium includes many types, and Plasmodium falciparum (P. falciparum), Plasmodium vivax (P. vivax), Plasmodium ovale (P. ovale), and Plasmodium malariae (P. malariae) mainly infect humans to cause clinical problems.

It was found in the present invention that, among these malaria plasmodiums, a clear difference in the aforementioned height of frequency peak part exists between Plasmodium falciparum and Plasmodium vivax, and the frequency of Plasmodium vivax tends to be higher than the frequency of Plasmodium falciparum, as shown in the below-mentioned Experimental Example 3 and the frequency distribution charts of Fig. 5. In a preferred embodiment of the present invention, therefore, a malaria plasmodium distinguishing part may be further provided in the analysis part. In the malaria plasmodium distinguishing part, the type of malaria plasmodium can be distinguished based on the height of the frequency peak part in the aforementioned volume range unique to malaria plasmodium. In the example of Fig. 1, the malaria plasmodium distinguishing part is contained in the condition determining part 431 in the determining part 430.

### [correction of malaria determination by platelet analysis]

In the WBC chamber, platelets are also counted. When platelets aggregate, they may become one large particle equivalent to malaria plasmodium. In the present invention, note has been taken of the possibility that the frequency of platelet aggregate is included in the frequency peak part within the aforementioned volume range unique to malaria plasmodium. By analyzing the platelets, it is possible to correct the height of the frequency peak part by subtracting the frequency of the aggregated platelets from the aforementioned height of the frequency peak part.

### [distinction between malaria positivity and dengue virus positivity]

In the present invention, note has been taken of the facts that, also in the case of dengue virus positivity, the frequency distribution of leukocyte changes and the frequency peak part is generated near the aforementioned volume range unique to malaria plasmodium, as shown in Experimental Example 4 described later and the frequency distribution chart of Fig. 6. The dengue virus is a minute particle that cannot be detected by the particle counting mechanism. As shown in the frequency distribution chart of Fig. 6, a frequency peak part may be generated near the aforementioned volume range unique to malaria plasmodium. However, as is clear from the frequency distribution chart of Fig. 3 (malaria positivity) and the frequency distribution chart of Fig. 6 (dengue virus positivity), malaria positivity and dengue virus positivity are markedly different from each other in the width of the frequency peak part. In malaria positivity, the frequency peak part is comparatively sharp, whereas the shape of the frequency peak part is clearly wide in dengue virus positivity.

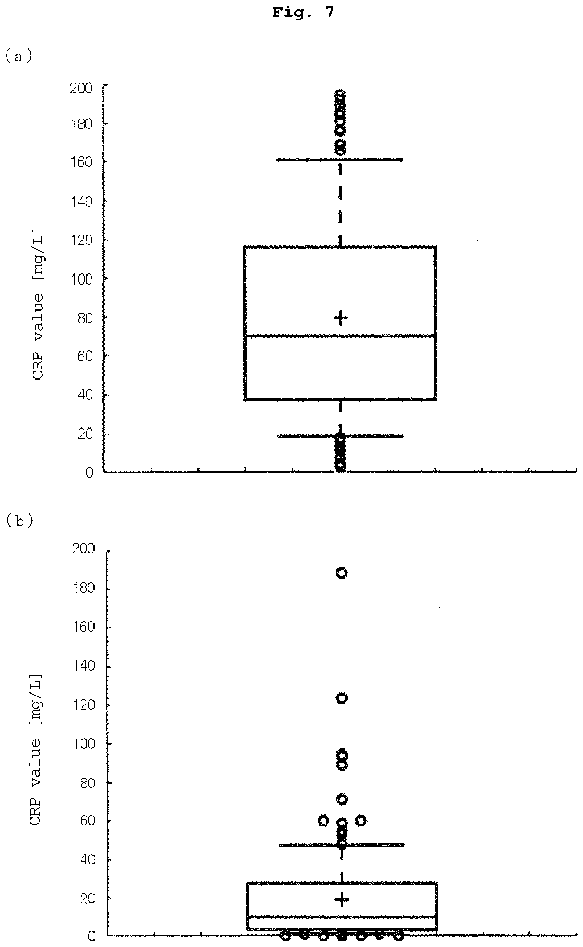

In the present invention, moreover, note has been taken of the markedly different CRP values between malaria positivity and dengue virus positivity. As shown in Experimental Example 5 described later and the box plot frequency distribution chart of Fig. 7, the range of the CRP value in the center part (box part in Fig. 7) of the distribution containing 50% of the number of specimens is larger in malaria positivity than in dengue virus positivity.

From the above aspects, it is also possible to provide a malaria-dengue distinguishing part configured to judge which of the likelihood of malaria positivity and the likelihood of dengue virus positivity is high based on the width of the frequency peak part, or based on the width of the frequency peak part and the aforementioned CRP value.

### [computer program by the present invention]

The computer program of the present invention (hereinafter to be also referred to as the program) is explained below. The program may be provided after recording on a computer-readable medium, or may be provided through a network from other computer or external storage device.

The program is preferably used for malaria determination in the control part of the blood analysis device of the present invention. The device for each measurement in the program and the processing for analysis are as mentioned above, and the measurement, analysis of measured values, flow of malaria determination, and the threshold value for determination of CRP value are as described above.

The program is a computer program that allows a computer to perform:
a step of receiving measured test values of a blood specimen to be analyzed (steps S1 - step S4 in the below-mentioned Example);
a step of determining whether the aforementioned blood specimen has a likelihood of being malaria positive based on a measured test value for a specific blood test item other than the CRP value and/or a measured test value regarding the CRP value (steps S5 - step S7 in the below-mentioned Example).
The aforementioned measured test values are measured values of respective predetermined blood test items including a CRP value and a counted value of blood cells. In addition, the specific blood test item is a blood test item related to malaria determination.

In a preferred embodiment, the program is a computer program that allows a computer to perform the following steps S1 - S7. For explanation, the symbols in the following steps are the symbols of respective parts in the device exemplified in Fig. 1.

### (step S1)

A step of receiving a volume measured value of each particle from a first particle measurement part (WBC chamber) 11 that measures a volume of each particle (particle containing leukocyte) in a sample liquid (optionally diluted in preferred embodiment) resulting from a hemolysis treatment of a blood specimen X1.

### (step S2)

A step of calculating a volume frequency distribution of the aforementioned each particle based on the volume measured value thereof.

### (step S3)

A step of receiving a CRP parameter value from a CRP measurement part (CRP chamber) 13 that measures the CRP parameter value corresponding to a CRP value of the blood specimen X1.

### (step S4)

A step of determining the CRP value based on the CRP parameter value.

### (step S5)

A step of determining whether a frequency peak part exists within a volume range unique to malaria plasmodium in the aforementioned volume frequency distribution.

### (step S6)

a step of determining whether the CRP value determined above is not less than a threshold value determined in advance,

### (step S7)

A step of determining that the aforementioned blood specimen has a high likelihood of being malaria positive when the aforementioned frequency peak part exists and when the CRP value determined above is not less than the threshold value.

By the computer program that allows a computer to perform the above steps, malaria determination can be performed with accuracy and reliability higher than those of conventional art.

In a preferred embodiment of the program, a malaria plasmodium distinction step is further provided, and the type of malaria plasmodium (Plasmodium vivax, Plasmodium falciparum) may be distinguished based on the height of the frequency peak part in the aforementioned volume range unique to malaria plasmodium.

In a preferred embodiment of the program, a step of calculating the volume frequency distribution of particles containing platelets and received from a second particle measurement part is further provided, and the volume and frequency of an agglomerate formed by aggregation of platelets may be calculated. As mentioned above, a step of correcting the height of the frequency peak part unique to malaria plasmodium according the frequency distribution of an agglomerate formed by aggregation of platelets and correcting the determination relating to malaria positivity may be provided.

In a preferred embodiment of the program, moreover, a malaria-dengue distinguishing part is further provided and which of the likelihood of malaria positivity and the likelihood of dengue virus positivity is high may be judged based on the width of the frequency peak part, or based on the width of the frequency peak part and the aforementioned CRP value.

### [Experimental Example 1]

In this Experimental Example, a blood specimen (specimen number 147) confirmed to be malaria positive by a morphological diagnostic method using a blood smear was subjected to a hemolysis treatment, particles (leukocytes) were counted, and the frequency distribution of the particles was calculated from the measurement results. The graph of Fig. 3 shows the volume frequency distribution charts of respective blood specimens (range of channels 0 - 100) superimposed in one graph.

As is clear from the graph of Fig. 3, it was found that the frequency peak part unique to malaria plasmodium appear in channels 17 - 23.

### [Experimental Example 2]

The malaria-positive blood specimens used in Experimental Example 1 were subjected to counting of particles (leukocytes), and measurement of CRP value, and the height of the frequency peak part unique to malaria plasmodium that appeared in channels 17 - 23 of the volume frequency distribution chart was associated with the CRP value for each blood specimen. The height of the frequency peak part was determined by a difference in height based on the frequency of the bottom of the valley part (peak to valley), the valley part being adjacent to the frequency peak part on the side with a larger volume. The respective blood specimens were plotted on a scatter diagram with the height of frequency peak part on the horizontal axis, and the CRP value on the vertical axis.

The scatter diagram of the results is shown in the graph of Fig. 4. The vertical broken line in the graph is a line indicating that the height of the frequency peak part is 1, and the horizontal broken line in the graph is a line indicating that the CRP value is 26 mg/L.

In the scatter diagram of Fig. 4, not less than 94% of the malaria positive samples were included in the region where the height of the frequency peak part in the volume frequency distribution of particles containing leukocyte and malaria plasmodium is one or more, and where the CRP value is not less than 26 mg/L. It was found that the determination of malaria positivity can be performed accurately by comparatively simple measurements of particle count of blood specimens and CRP value measurement.

### [Experimental Example 3]

In this Experimental Example, a blood specimen falci confirmed to have been infected with Plasmodium falciparum by a RDT (Rapid Diagnostic. Test) method and a blood specimen vivax confirmed to have been infected with Plasmodium vivax were each subjected to a hemolysis treatment in the same manner as in Experimental Example 1, particles (leukocytes) were counted by the same blood analysis device, and the frequency distribution of the particles was calculated from the measurement results. The graph of Fig. 5 shows the volume frequency distribution charts of respective blood specimens (range of channels 1 - 254) superimposed in one graph. In the graph of Fig. 5, the thick line shows the volume frequency distribution chart of particles contained in the blood specimen vivax, and the thin line is the volume frequency distribution chart of particles contained in the blood specimen falci.

As is clear from the graph of Fig. 5, while the frequency peak part unique to malaria plasmodium appears near channel 20 in all blood specimens, it was found that the height of the frequency peak part (peak to valley) is greater in blood specimen vivax than in blood specimen falci, and the height of the frequency peak part based on frequency 0 is also greater in blood specimen vivax than in blood specimen falci.

### [Experimental Example 4]

In this Experimental Example, a blood specimen (specimen number 170) confirmed to be dengue virus positive by an enzyme immunoassay (ELISA method) was subjected to a hemolysis treatment, particles (leukocytes) were counted, and the frequency distribution of the particles was calculated from the measurement results. The graph of Fig. 6 shows the representative volume frequency distribution charts of respective blood specimens (range of channels 0 - 100) superimposed in one graph.

As is clear from the graph of Fig. 6, a frequency peak part appeared near similar channels of the peak.

### [Experimental Example 5]

In this Experimental Example, each CRP value of blood specimens confirmed to be malaria positive as in Experimental Example 1, and blood specimens confirmed to be dengue virus positive as in Experimental Example 4 was measured.

Fig. 7(a) is a box plot showing the CRP values of malaria-positive blood specimens, and Fig. 7(b) is a box plot showing the CRP values of dengue fever-positive blood specimens. In each box plot, the horizontal line on the lower side of the box is a line of 9% from the smallest distribution of CRP value, the lower limit of the box is a 25% line, the horizontal line in the box is an average value, the upper limit of the box is a 75% line, and the horizontal line on the upper side of the box is a 91% line. The plots of not more than the 9% line and the plots of not less than the 91% line are shown, and plots with the same CRP value are horizontally shown side by side.

From the graphs of Fig. 7(a) and Fig. 7(b), it was found that the CRP value varies greatly between malaria positivity and dengue virus positivity, and the CRP value is higher in malaria positivity than in dengue virus positivity.

### (embodiment 1 of malaria determination using artificial intelligence)

In a preferred embodiment 1 of the device, the blood test items for malaria determination are selected by artificial intelligence. In this embodiment, the determining part 430 in Fig. 1 uses an important test item related to malaria positivity (or malaria determination) which is specified from predetermined blood test items (various blood test items) by the first learned model M1 described later. In addition, the determining part 430 is configured to perform malaria determination based on the measured test value of the specified important test item.

### (first learned model M1)

The first learned model M1 is formed by machine learning by artificial intelligence which of the predetermined blood test items is related to malaria positivity by using the following data (a1) and (b1) as the teaching data.
(a1) measured test value (A1) obtained for the aforementioned predetermined blood test item by using blood specimen (A) known to be malaria positive as a measuring object, and information (A2) that the measured test value (A1) is of a malaria positive blood specimen
(b1) measured test value (B1) obtained for the aforementioned predetermined blood test item by using blood specimen (B) known to be malaria negative as a measuring object, and information (B2) that the measured test value (B1) is of a malaria negative blood specimen.

The blood test item(s) specified by the first learned model M1 is/are important test item(s) (i.e., blood test item(s) related to malaria positivity). In this embodiment 1, the important test items may include all blood test items related even a little with malaria positivity.

The important test item(s) specified by the first learned model M1 may be, for example, held in the reference data holding part 432 in Fig. 1, or incorporated into the condition determining part 431 as determination condition(s). The condition determining part 431 refers to the important test item(s) held in the reference data holding part 432, receives the measured test value(s) relating to the important test item(s) from the measured value receiving part 410 or measured value processing part 420, and performs condition determination determined in advance. The determining part 433 receives the results of condition determination by the condition determining part 431 and performs malaria determination. That is, in this embodiment 1, the important test item(s) is/are specified in advance by a learned model, and the malaria determination is performed using measured test value(s) of the important test item(s). The same applies to other embodiments below.

In the present invention, the artificial intelligence and the learned model are preferably constructed by a computer different from the device (neuro computer configured to meet the principles of artificial intelligence, and the like). It may also be provided within the device.

The artificial intelligence and the learned model that are constructed by a computer different from the device may be connected to the device through a communication line.

In addition, malaria determination of blood specimens that underwent malaria determination by the device (or blood specimen separately collected from patients from which the blood specimens were collected) may be performed more accurately by a conventional precise testing method, the test value(s) measured by the device and the malaria determination results by the conventional test method may be fed back to the artificial intelligence as teaching data, and the learning state of the learned model may be updated further.

### (important test item specified by learned model M1)

As the important test items specified by learned model M1, for example, the following items (e1) - (e4) can be mentioned.

(e1) Whether or not a frequency peak exists in the volume range of 25 fL - 45 fL in the volume frequency distribution of leukocytes in a blood specimen to be analyzed. In the following, the frequency peak is to be also referred to as "L1 peak in volume frequency distribution of leukocytes". The L1 peak in the volume frequency distribution of leukocytes is, for example, as explained above using Fig. 3, and shown as "frequency peak part caused by malaria plasmodium" in the frequency distribution chart of Fig. 5.

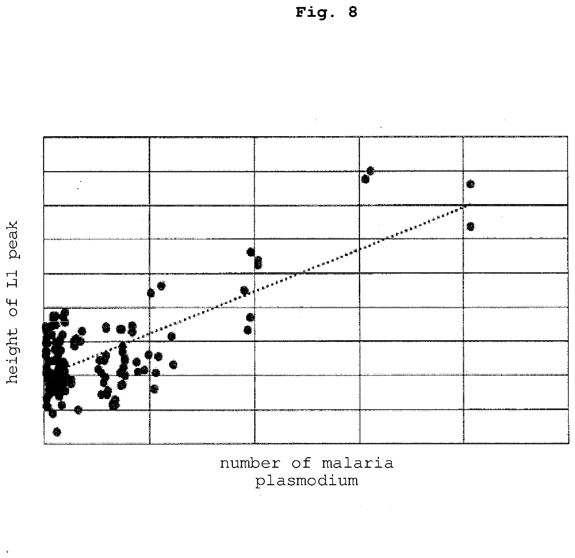

According to the study by the present inventors, a clear correlation as shown in the graph chart of Fig. 8 exists between the height of L1 peak in the volume frequency distribution of leukocytes and the number of malaria plasmodiums contained per unit volume of malaria-positive blood. The height of L1 peak may be, as explained above by using Fig. 3, a value determined by peak to valley, height based on zero frequency (= frequency value itself of frequency peak part) or the like.

(e2) Leukocyte count of blood specimen to be analyzed. The blood test items include an item for measuring the number of leukocytes in a specific volume of blood (1 µL or the like), without classifying leukocytes in detail. It is known that the leukocyte count shows an abnormal value (increase or decrease) in malaria positive blood, which is consistent with the conventional findings.

(e3) Platelet count of blood specimen to be analyzed. The blood test items include an item for measuring the number of platelets in a specific volume of blood (1 µL or the like). It is known that the platelet count shows an abnormal value (decrease) in malaria positive blood, which is consistent with the conventional findings.

(e4) CRP value of blood specimen to be analyzed. The blood test items include an item for measuring the amount of CRP (C-reactive protein) in a specific volume of serum (1 µL or the like). The present invention proposes for the first time the use of CRP value for malaria determination. As clarified by the present invention, the CRP value is correlated with malaria positivity, and shows an abnormal value (increase) in malaria positive blood.

The important test items may contain one or more items selected from the group consisting of the aforementioned items (e1) - (e4). To obtain more accurate determination results, for example, malaria determination is preferably performed using the aforementioned items (e1) and (e2), or the aforementioned items (e1) and (e3), or the aforementioned items (e1) and (e4), or the aforementioned items (e2) and (e3), or the aforementioned items (e2) and (e4), or the aforementioned items (e3) and (e4), or the aforementioned items (e1), (e2), and (e3), or the aforementioned items (e1), (e2), and (e4), or the aforementioned items (e1), (e3), and (e4), or the aforementioned items (e2), (e3), and (e4), or further, all the aforementioned items (e1) - (e4).

### (modified embodiment of embodiment 1 of malaria determination using artificial intelligence)

In this modified embodiment, the aforementioned determining part uses an important test item related to malaria positivity at a level not less than a predetermined degree which is specified from the above-mentioned blood test items by a learned model M11 obtained by modifying the first learned model. The determining part is configured to determine whether the aforementioned blood specimen has a likelihood of being malaria positive based on the measured test value of the specified important test item.

Also in this modified embodiment, like the aforementioned embodiment 1, the important test item(s) specified by the first learned model M1 may be, for example, held in the reference data holding part 432 in Fig. 1. The condition determining part 431 refers to the important test item(s) held in the reference data holding part 432, receives the measured test value(s) relating to the important test item(s) from the measured value receiving part 410 or measured value processing part 420, and performs arithmetic operation as to whether they meet the condition(s) determined in advance.

### (learned model M11 obtained by modifying the first learned model M1)

This learned model M11 is formed by machine learning by artificial intelligence which of the predetermined blood test items is related to malaria positivity in what degree by using the following data (a11) and (b11) as the teaching data.
(a11) measured test value (A1) obtained for the aforementioned predetermined blood test item by using blood specimen (A) known to be malaria positive as a measuring object, and information (A2) that the measured test value (A1) is of a malaria positive blood specimen
(b11)measured test value (B1) obtained for the aforementioned predetermined blood test item by using blood specimen (B) known to be malaria negative as a measuring object, and information (B2) that the measured test value (B1) is of a malaria negative blood specimen.

In this modified embodiment, the important test items include only the blood test items with higher relevance to malaria positivity than the predetermined degree and does not include blood test items with small relevance that can be ignored. On the other hand, the important test items in the aforementioned embodiment 1 include all blood test items judged to have relevance even when the relevance is small.

The determining part is configured to use an important test item with higher relevance to malaria positivity than the predetermined degree which is specified from the predetermined blood test items by the modified learned model M11, and perform malaria determination of blood specimen based on the measured test value of the specified important test item. For example, among all blood test items having even a small relevance to malaria positivity, the top n item(s) in descending order of relevance (e.g., n= about 1 - 5, preferably n= about 2 - 4) may be selected as important test item(s). Alternatively, the relevance may be expressed by a weighting coefficient, and only the item(s) with weighting coefficient(s) not less than a given value (with high relevance) may be selected as important test item(s).

The important test item(s) specified by modified learned model M11 may be similar to the aforementioned items (e1) - (e4).

### (embodiment 2 of malaria determination using artificial intelligence)

In a preferred embodiment 2 of the device, the blood test items for malaria determination are selected by artificial intelligence, and the degree of relevance of each of the aforementioned blood test items to malaria positivity is specified by artificial intelligence. In this embodiment 2, the determining part 430 in Fig. 1 uses a blood test item (important test item) related to malaria positivity (or malaria determination), which item is specified by the second learned model M2 described later, and also uses the degree of relevance of each of the blood test items to malaria positivity (or malaria determination), which degree is specified by the second learned model M2. As used herein, to specify the degree of relevance of each of the blood test items means to specify the weight corresponding to the degree of relevance to malaria positivity (or malaria determination) for each blood test item. The determining part 430 is configured to perform malaria determination based on the measured test value of the specified important test item and the aforementioned specified degree (weight) of relevance.

In this embodiment 2, the important test item(s) and degree(s) (weight(s)) of relevance, which are specified by the second learned model M2, may be held in the reference data holding part 432 in Fig. 1, or incorporated into the condition determining part 431 as determination conditions.

For example, the condition determining part 431 first refers to the important test item(s) held in the reference data holding part 432, receives the measured test value(s) relating to the important test item(s) from the measured value receiving part 410 or measured value processing part 420. Then, the condition determining part 431 performs arithmetic operation as to whether they meet the condition(s) determined by incorporating the weight for each important test item.

### (second learned model M2)

The second learned model M2 may be the same as the aforementioned learned model M11. Even in an embodiment utilizing the same learned model, the modified embodiment in the aforementioned embodiment 1 aims to eliminate unnecessary blood test items, and the obtained important test items were used under equivalent conditions. In contrast, embodiment 2 aims to obtain important test item(s) and a weight for each important test item and, during malaria determination, the determination is performed by attaching a weight to each measured test value relating to each important test item.

In this embodiment 2, the degree of relevance of each important test item as a weighting coefficient is added to the measured test value of the important test item specified by the second learned model M2, a criterion formula for malaria determination is constituted, and the malaria determination may be performed according to the value of the criterion formula. For example, a threshold value or the presence or absence of a state is set in advance with respect to the value of the criterion formula, and the malaria determination may be performed according to whether the value of the criterion formula is not less than or less than the threshold value, or meets the presence or absence of the state. the criterion formula, threshold value, the presence or absence of the state, and the like may be held in the condition determining part 431 or reference data holding part 432 in Fig. 1.

When a plurality of important test items are involved, a criterion formula is constituted by adding a weighting coefficient corresponding to each measured test value of the plurality of important test items, and the determination may be performed according to the value (determination value) indicated by the criterion formula. The criterion formula may be a formula obtained by (f1) adding respective weighting coefficients to measured test values of specified important test items according to a predetermined arithmetic operation (sum, difference, quotient, product and the like), and (f2) combining the measured test values accorded with the aforementioned weighting coefficients as a result of the predetermined arithmetic operation (sum, difference, quotient, product).

From the viewpoint that the arithmetic operation is simple and the influence of each item is easy to understand for the designer, operator, user, and the like of the device, the aforementioned criterion formula is preferably one obtained by (f1) adding respective weighting coefficients in the form of a product to the measured test values of specified important test items, and (f2) combining the products of the weighting coefficients and the measured test values in the form of a sum.

That is, this embodiment 2 may be a device configured to execute
a step of arithmetically processing a product (p1×Q1, p2×Q2, p3×Q3, ..., pk×Qk) of each important test item (Q1, Q2, Q3, ..., Qk) among the important test items and weighting coefficients (p1, p2, p3, ..., pk) corresponding to each important test item wherein k is an integer of one or more, which is about 2 - 4 in a preferred embodiment),
a step of arithmetically processing, as a determination value, the sum (p1×Q1+p2×Q2+p3×Q3+ ... +pk×Qk) of respective items (p1×Q1, p2×Q2, p3×Q3, ..., pk×Qk) arithmetically processed in the aforementioned step, and
a step of determining that the blood specimen has a high likelihood of being malaria positive by comparison of the aforementioned determination value and the threshold value determined in advance.

Also, it may be a computer program configured to allow a computer to execute the aforementioned steps, or a blood analysis method for performing these steps (malaria determination method, method for assuming malaria positivity).

When the test item has greater relevance to malaria positivity, measured test value of the important test item exerts a greater influence on the malaria determination. The aforementioned criterion formula (p1×Q1+p2×Q2+p3×Q3+... +pk×Qk) or a value (determination value) thereof comprehensively expresses numerically an influence of changes in the measured test values of respective important test items. A higher value of weighting coefficient (p1, p2, p3,..., pk) is accorded to an important test item that exerts a greater influence on the malaria determination. In other words, an important test item accorded with a higher weighting coefficient has a greater influence on malaria determination.

The important test item may be in a plurality. When the important test item is in a plurality, for example, a predetermined number (e.g., about 1 - 5, preferably about 2 - 4) of blood test items may be adopted as important test items starting from the one having the largest weighting coefficient.

When a plurality of important test items are involved, malaria determination can be performed more accurately by using the weighting coefficient corresponding to each important test item.

In addition, predetermined arithmetic operation, threshold value and the like may be set and important test items may be adopted from many blood test items.

The aforementioned learned models (M1, M11, M2) can be constructed by giving the aforementioned teaching data to known artificial intelligence such as neural network (particularly, configured to perform deep learning) and the like, thereby allowing the artificial intelligence to perform machine learning. The operation of giving the aforementioned teaching data to artificial intelligence to allow for machine learning, and specifying important test items (and degrees of relevance to malaria positivity) from the predetermined blood test items corresponds to classification (distinction).

The predetermined blood test items to be the target of machine learning by artificial intelligence include hematological test items, biochemical test items, and immunological test items, such as CRP value, volume frequency distribution of blood cells to be measured, L1 peak in volume frequency distribution of leukocyte, platelet count, leukocyte count, erythrocyte count, hemoglobin content, hematocrit value, mean corpuscular volume, mean corpuscular hemoglobin, mean corpuscular hemoglobin concentration, red blood cell distribution width-average deviation, red blood cell distribution width, mean platelet volume, platelet volume, platelet distribution width, platelet-large cell ratio, large platelet count, lymphocyte count, monocyte count, neutrophil count, eosinophils count, basophil count, lymphocyte ratio, monocyte ratio, neutrophil ratio, eosinophils ratio, basophil ratio, atypical lymphocyte, large juvenile cell, various blood counts measured by the aforementioned particle counting mechanism, and the like. Artificial intelligence specifies important test items from these test items. The blood test items may be determined according to the blood analysis mechanism provided in the blood analysis device. For example, when the blood analysis device does not include a CRP measurement part, the important test items may not include the CRP value. A blood analysis device provided with an erythrocyte measurement part, a leukocyte measurement part, and a CRP measurement part is more preferred, and it is preferable to include a CRP value as an important test item.

As the weighting coefficient given by the second learned model, the degree of relevance (e.g., numerical value (proportion) etc. according to each degree of relevance when the total is 1, 100, etc.) may be adopted as it is, or predetermined arithmetic operation may be performed on the degree of relevance to obtain a weighting coefficient.

In the aforementioned embodiment, the case where a plurality of important test items are adopted has been explained. When the degree of relevance of a certain one important test item is particularly larger than the degree of relevance of other important test items, only the important test item with a particularly large degree of relevance may be adopted from the specified plural important test items. In this case, a weighting coefficient may be set only on the adopted one important test item.

In addition, a predetermined arithmetic operation, a threshold value or the like may be set and a criteria for re-adopting an important test item may be incorporated into the second learned model.

### (other embodiment of malaria determination using artificial intelligence)

In the construction of a learned model, the aforementioned embodiment 1 (or modified embodiment of embodiment 1) and embodiment 2 may be combined in two steps to construct a third learned model M3. That is, the third learned model M3 is formed by machine learning by artificial intelligence which of the specified important test item is related to malaria positivity in what degree by using the following data (a3) and (b3) as the teaching data.
(a3) measured test value (A11) obtained for the important test item specified by the aforementioned first learned model M1 (or M11) by using blood specimen (A) known to be malaria positive as a measuring object, and information (A21) that the measured test value (A11) is of a malaria positive blood specimen
(b3) measured test value (B11) obtained for the important test item specified by the aforementioned first learned model M1 (or M11) by using blood specimen (B) known to be malaria negative as a measuring object, and information (B21) that the measured test value (B11) is of a malaria negative blood specimen

In the third learned model M3, blood test items have been specified in advance by the first learned model M1 and the items not contributing to the determination have been eliminated during construction of the second learned model M2. As a result, the weight obtained by the third learned model M3 (which of the specified important test item is related at what degree to malaria positivity) can sometimes afford malaria determination with high accuracy than the weight obtained by the second learned model M2 alone.

In contrast, when the aforementioned embodiment 2 is performed singly, since important test item(s) and weight(s) thereof are specified from all blood test items, the accuracy of the malaria determination may decrease due to a blood test item with a minimal relevance. This is considered to be caused by the fact that a blood test item with minimal relevance is not adopted as an important test item. In such a case, the accuracy of malaria determination may be improved by applying principal component analysis or sparse optimization to the degree of relevance.

In the aforementioned embodiment, the threshold value for the determination of CRP value was obtained in advance by experiments and stored in the reference data holding part 432. The threshold value for the determination of the CRP value may also be obtained by a learned model constructed by machine learning. In addition, in each embodiment of malaria determination using the aforementioned artificial intelligence, each threshold value used for malaria determination may also be obtained similarly by constructing various learned models.

### (computer program (1) for malaria determination using artificial intelligence)

The determination step in the aforementioned computer program may be configured to include a step of using an important test item related to malaria positivity and specified by the aforementioned first learned model M1 or M11 and performing malaria determination of blood specimen based on the measured test value of the aforementioned important test item.

The important test item and malaria determination are as described in the explanation of the device.

### (computer program (2) for malaria determination using artificial intelligence)

The determination step in the aforementioned computer program may be configured to include a step of using a degree of relevance to each of predetermined blood test items for malaria positivity which is specified by the above-mentioned second learned model M2 to determine whether the aforementioned blood specimen has a likelihood of being malaria positive based on the measured test value of the above-mentioned blood test item and the aforementioned specified degree of relevance.

The important test item and malaria determination are as described in the explanation of the device.

The computer program of the present invention is configured to allow a computer to perform various embodiments of the determination action of the aforementioned device.

### (method of the present invention)

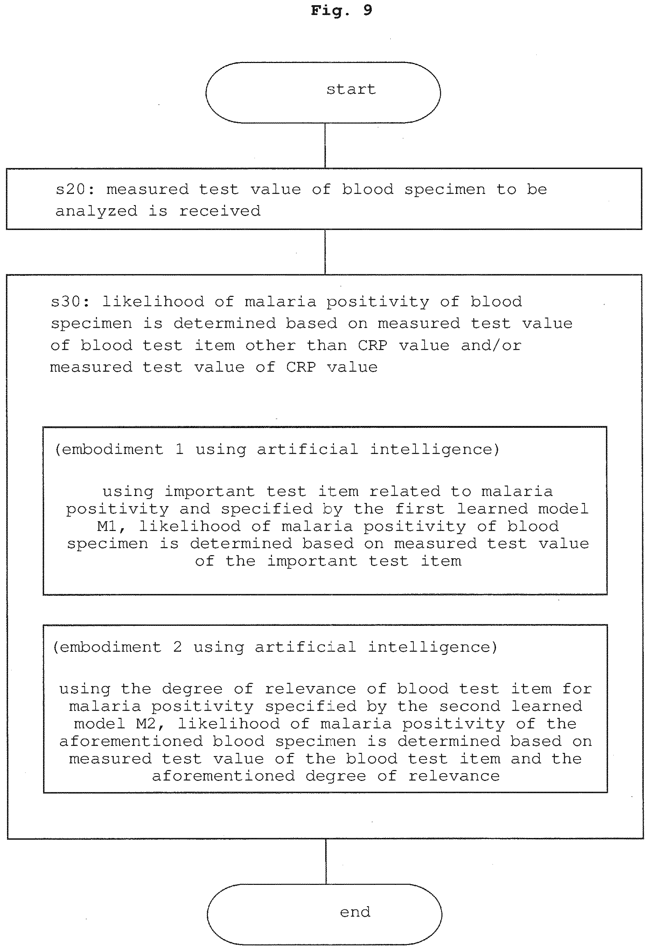

The blood analysis method of the present invention is a method for performing malaria determination. The method has a step s20 of preparing measured test values of a blood specimen to be analyzed, as shown in the flowchart of Fig. 9. The measured test values here are measured values of respective predetermined blood test items including a CRP value and a counted value of blood cells. The method has a step s30 of determining whether the aforementioned blood specimen has a likelihood of being malaria positive based on a measured test value for a blood test item other than the CRP value and/or a measured test value regarding the CRP value.

The important test item, malaria determination are as described in the explanation of the device.

### (embodiment 1 of malaria determination in blood analysis method using artificial intelligence)

In this embodiment 1, the aforementioned determination step s30 uses an important test item related to malaria positivity which is specified from the above-mentioned blood test items by the above-mentioned first learned model M1. It has a step of determining whether the aforementioned blood specimen has a likelihood of being malaria positive based on the measured test value of the important test item.

### (embodiment 2 of malaria determination in blood analysis method using artificial intelligence)

In this embodiment 2, the aforementioned determination step s30 uses a degree of relevance to each of predetermined blood test items for malaria positivity which is specified by the above-mentioned second learned model M2. In addition, it has a step of determining whether the aforementioned blood specimen has a likelihood of being malaria positive based on the measured test value of the above-mentioned blood test item and the aforementioned specified degree of relevance.

The description of the contents of operation of each part for malaria determination in the description of the device in the above is also a description of the computer program of the present invention and also a description of the blood analysis method of the present invention.

### [Industrial Applicability]

According to the present invention, a blood analysis device capable of performing malaria determination even though the constitution is inexpensive can be provided at a low cost. According to the present invention, moreover, a computer program capable of having a computer perform malaria determination more preferably and a blood analysis method capable of performing malaria determination more preferably can be provided.

This application is based on a patent application No. 2018-246186 filed in Japan (filing date: December 27, 2018), the contents of which are incorporated in full herein.

### [Explanation of Symbols]

- M1: blood specimen
- 10: measuring mechanism part
- 11: first particle measurement part
- 12: second particle measurement part
- 13: CRP measurement part
- 14: dispensing mechanism
- 20: control part
- 30: mechanism control part
- 40: analysis part
- 410: measured value receiving part (including volume value receiving part and CRP value receiving part)
- 420: measured value processing part
- 421: frequency distribution calculating part
- 423: CRP value determining part
- 430: determining part
- 440: determination result output part

## Claims

1. A blood analysis device comprising a measured value receiving part (410) and a determining part (430), wherein
the measured value receiving part (410) receives measured test values of a blood specimen (M1) to be analyzed,
the measured test values are measured values of respective predetermined blood test items including a CRP value based on a CRP parameter value and a counted value of blood cells,
the measured value receiving part (410) has a CRP value receiving part for receiving a CRP parameter value in a sample liquid comprising the blood specimen (M1), and
the determining part (430) determines whether the blood specimen (M1) has a likelihood of being malaria positive based on a measured test value of at least one counted value of blood cells and a specific measured test value regarding the CRP value.

2. The blood analysis device according to claim 1, wherein the measured value receiving part (410) has a volume value receiving part that receives a volume measured value of a particle in the sample liquid comprising the blood specimen (M1), and
the determining part (430) determines that the blood specimen (M1) has a high likelihood of being malaria positive when a frequency peak part exists in a predetermined volume range in a volume frequency distribution based on the volume measured value, or when the CRP value based on the CRP parameter value is not less than a threshold value determined in advance.

3. The blood analysis device according to claim 2, further comprising a leukocyte measurement chamber as a first particle measurement part (11), wherein
in the leukocyte measurement chamber, a blood specimen (M1) to be supplied is subjected to a hemolysis treatment to lyse erythrocytes and diluted to form the sample liquid, and a volume of each of leukocytes and particles other than leukocytes in the sample liquid is measured, and
the volume value receiving part receives the volume measured value from the first particle measurement part (11).

4. The blood analysis device according to claim 2 or 3, further comprising a CRP measurement part (13) that measures the CRP parameter value, wherein
The CRP value receiving part receives the CRP parameter value from the CRP measurement part (13).

5. The blood analysis device according to any one of claims 2 to 4, wherein the determining part (430) determines that the blood specimen (M1) has a high likelihood of being malaria positive when the frequency peak part exists in the predetermined volume range in the volume frequency distribution based on the volume measured value, and when the CRP value based on the CRP parameter value is not less than the threshold value determined in advance.

6. The blood analysis device according to claim 1, wherein the determining part (430) is configured to use an important test item related to malaria positivity which is specified from predetermined blood test items by the following first learned model, and determine whether the blood specimen (M1) has a likelihood of being malaria positive based on a measured test value of the important test item,
wherein the first learned model is formed by machine learning which of the predetermined blood test items is related to malaria positivity by using, as teacher data, measured test value (A1) obtained for the predetermined blood test item by using blood specimen (A) known to be malaria positive as a measuring object, and information (A2) that the measured test value (A1) is of a malaria positive blood specimen, and measured test value (B1) obtained for the predetermined blood test item by using blood specimen (B) known to be malaria negative as a measuring object, and information (B2) that the measured test value (B1) is of a malaria negative blood specimen.

7. The blood analysis device according to claim 6, wherein the important test item includes one or more selected from the group consisting of
whether a frequency peak exists in a volume range of 25fL to 45fL in a volume frequency distribution of leukocytes in a blood specimen (M1) to be analyzed,
leukocyte count of a blood specimen (M1) to be analyzed,
platelet count of a blood specimen (M1) to be analyzed, and
CRP value of a blood specimen (M1) to be analyzed.

8. The blood analysis device according to claim 1, wherein the determining part (430) is configured to use a degree of relevance to each of predetermined blood test items for malaria positivity which is specified by the following second learned model (M2), and determine whether the blood specimen (M1) has a likelihood of being malaria positive based on a measured test value of the predetermined blood test item and the specified degree of relevance,
wherein the second learned model (M2) is formed by machine learning which of the predetermined blood test items is related to malaria positivity in what degree by using, as teacher data, measured test value (A1) obtained for the predetermined blood test item by using blood specimen (A) known to be malaria positive as a measuring object, and information (A2) that the measured test value (A1) is of a malaria positive blood specimen, and measured test value (B1) obtained for the predetermined blood test item by using blood specimen (B) known to be malaria negative as a measuring object, and information (B2) that the measured test value (B1) is of a malaria negative blood specimen.

9. A computer program that allows a computer to perform a step of receiving measured test values of a blood specimen (M1) to be analyzed, wherein the measured test values are measured values of respective predetermined blood test items including a CRP value and a counted value of blood cells, and
A step of determining whether the blood specimen (M1) has a likelihood of being malaria positive based on a measured test value for at least one counted value of blood cells and a measured test value regarding the CRP value.

10. The computer program according to claim 9, wherein the computer performs a step of receiving a volume measured value of a particle in the sample liquid comprising the blood specimen (M1),
a step of receiving a CRP parameter value in the sample liquid,
a step of determining whether a frequency peak part exists within a volume range unique to malaria plasmodium in the volume frequency distribution based on the volume measured value,
a step of determining whether the CRP value based on the CRP parameter value is not less than a threshold value determined in advance, and
a step of determining that the blood specimen (M1) has a high likelihood of being malaria positive when the frequency peak part exists or when the CRP value is not less than the threshold value.

11. The computer program according to claim 10, further comprising a computer program that allows a computer to perform a step of determining that the blood specimen (M1) has a high likelihood of being malaria positive when the frequency peak part exists and the CRP value is not less than the threshold value.

12. The computer program according to claim 9, wherein the determination step comprises a step of using an important test item related to malaria positivity which is specified from predetermined blood test items by the following first learned model, and determining whether the blood specimen (M1) has a likelihood of being malaria positive based on a measured test value of the important test item,
wherein the first learned model is formed by machine learning which of the predetermined blood test items is related to malaria positivity by using, as teacher data, measured test value (A1) obtained for the predetermined blood test item by using blood specimen (A) known to be malaria positive as a measuring object, and information (A2) that the measured test value (A1) is of a malaria positive blood specimen, and measured test value (B1) obtained for the predetermined blood test item by using blood specimen (B) known to be malaria negative as a measuring object, and information (B2) that the measured test value (B1) is of a malaria negative blood specimen.

13. The computer program according to claim 9, wherein the determination step comprises a step of using a degree of relevance to each of predetermined blood test items for malaria positivity which is specified by the following second learned model (M2), and determining whether the blood specimen (M1) has a likelihood of being malaria positive based on a measured test value of the predetermined blood test item and the specified degree of relevance,
wherein the second learned model (M2) is formed by machine learning which of the predetermined blood test items is related to malaria positivity in what degree by using, as teacher data, measured test value (A1) obtained for the predetermined blood test item by using blood specimen (A) known to be malaria positive as a measuring object, and information (A2) that the measured test value (A1) is of a malaria positive blood specimen, and measured test value (B1) obtained for the predetermined blood test item by using blood specimen (B) known to be malaria negative as a measuring object, and information (B2) that the measured test value (B1) is of a malaria negative blood specimen.

14. A method for analyzing a blood specimen, comprising a step of preparing measured test values of a blood specimen (M1) to be analyzed, wherein the measured test values are measured values of respective predetermined blood test items including a CRP value and a counted value of blood cells, and
a step of determining whether the blood specimen (M1) has a likelihood of being malaria positive based on a measured test value for at least one counted value of blood cells and a measured test value regarding the CRP value.

15. The blood specimen analysis method according to claim 14, wherein the determination step comprises a step of using an important test item related to malaria positivity which is specified from predetermined blood test items by the following first learned model, and determining whether the blood specimen (M1) has a likelihood of being malaria positive based on a measured test value of the important test item,
wherein the first learned model is formed by machine learning which of the predetermined blood test items is related to malaria positivity by using, as teacher data, measured test value (A1) obtained for the predetermined blood test item by using blood specimen (A) known to be malaria positive as a measuring object, and information (A2) that the measured test value (A1) is of a malaria positive blood specimen, and measured test value (B1) obtained for the predetermined blood test item by using blood specimen (B) known to be malaria negative as a measuring object, and information (B2) that the measured test value (B1) is of a malaria negative blood specimen.

16. The blood specimen analysis method according to claim 14, wherein the determination step comprises a step of using a degree of relevance to each of predetermined blood test items for malaria positivity which is specified by the following second learned model (M2), and determining whether the blood specimen (M1) has a likelihood of being malaria positive based on a measured test value of the predetermined blood test item and the specified degree of relevance,
wherein the second learned model (M2) is formed by machine learning which of the predetermined blood test items is related to malaria positivity in what degree by using, as teacher data, measured test value (A1) obtained for the predetermined blood test item by using blood specimen (A) known to be malaria positive as a measuring object, and information (A2) that the measured test value (A1) is of a malaria positive blood specimen, and measured test value (B1) obtained by measuring for the predetermined blood test item by using blood specimen (B) known to be malaria negative as a measuring object, and information (B2) that the measured test value (B1) is of a malaria negative blood specimen.

## Patentansprüche

1. Blutanalysevorrichtung, umfassend einen Messwertempfangsteil (410) und einen Bestimmungsteil (430), wobei
der Messwertempfangsteil (410) gemessene Testwerte einer zu analysierenden Blutprobe (M1) empfängt,
die gemessenen Testwerte Messwerte jeweiliger zuvor festgelegter Bluttestkomponenten sind, einschließlich eines CRP-Wertes auf der Grundlage eines CRP-Parameterwertes und eines Zählwertes von Blutzellen,
der Messwertempfangsteil (410) einen CRP-Wertempfangsteil zum Empfangen eines CRP-Parameterwertes in einer Probenflüssigkeit, die die Blutprobe (M1) umfasst, aufweist, und
der Bestimmungsteil (430) auf der Grundlage eines gemessenen Testwertes von mindestens einem Zählwert von Blutzellen und eines spezifischen gemessenen Testwertes bezüglich des CRP-Wertes bestimmt, ob die Blutprobe (M1) eine Wahrscheinlichkeit besitzt, Malaria-positiv zu sein.

2. Blutanalysevorrichtung nach Anspruch 1, wobei der Messwertempfangsteil (410) einen Volumenwertempfangsteil aufweist, der einen Volumenmesswert eines Partikels in der Probenflüssigkeit, die die Blutprobe (M1) umfasst, empfängt, und
der Bestimmungsteil (430) bestimmt, dass die Blutprobe (M1) eine hohe Wahrscheinlichkeit besitzt, Malaria-positiv zu sein, wenn ein Frequenzspitzenteil in einem zuvor festgelegten Volumenbereich in einer Volumenfrequenzverteilung auf der Grundlage des Volumenmesswertes vorhanden ist, oder wenn der CRP-Wert auf der Grundlage des CRP-Parameterwertes nicht kleiner als ein im Voraus bestimmter Schwellenwert ist.

3. Blutanalysevorrichtung nach Anspruch 2, des Weiteren umfassend eine Leukozytenmesskammer als einen ersten Partikelmessteil (11), wobei
in der Leukozytenmesskammer eine zuzuführende Blutprobe (M1) einer Hämolysebehandlung unterzogen wird, um Erythrozyten zu lysieren, und verdünnt wird, um die Probenflüssigkeit zu bilden, und ein Volumen jedes von Leukozyten und anderer Partikel als Leukozyten in der Probenflüssigkeit gemessen wird, und
der Volumenwertempfangsteil den Volumenmesswert von dem ersten Partikelmessteil (11) empfängt.

4. Blutanalysevorrichtung nach Anspruch 2 oder 3, des Weiteren umfassend einen CRP-Messteil (13), der den CRP-Parameterwert misst, wobei
der CRP-Wertempfangsteil den CRP-Parameterwert von dem CRP-Messteil (13) empfängt.

5. Blutanalysevorrichtung nach einem der Ansprüche 2 bis 4, wobei der Bestimmungsteil (430) bestimmt, dass die Blutprobe (M1) eine hohe Wahrscheinlichkeit besitzt, Malaria-positiv zu sein, wenn der Frequenzspitzenteil in dem zuvor festgelegten Volumenbereich in der Volumenfrequenzverteilung auf der Grundlage des Volumenmesswertes vorhanden ist, und wenn der CRP-Wert auf der Grundlage des CRP-Parameterwertes nicht kleiner als der im Voraus bestimmter Schwellenwert ist.

6. Blutanalysevorrichtung nach Anspruch 1, wobei der Bestimmungsteil (430) dazu eingerichtet ist, eine wichtige Testkomponente bezüglich Malaria-Positivität, die anhand zuvor festgelegter Bluttestkomponenten durch das folgende erste erlernte Modell spezifiziert wird, zu verwenden und auf der Grundlage eines gemessenen Testwertes der wichtigen Testkomponente zu bestimmen, ob die Blutprobe (M1) eine Wahrscheinlichkeit besitzt, Malaria-positiv zu sein,
wobei das erste erlernte Modell durch maschinelles Lernen, welches der zuvor festgelegten Bluttestkomponenten mit Malaria-Positivität in Zusammenhang steht, gebildet wird, indem als Lehrerdaten verwendet werden der gemessene Testwert (A1), der für die zuvor festgelegte Bluttestkomponente unter Verwendung einer als Malaria-positiv bekannten Blutprobe (A) als ein Messobjekt erhalten wurde, und die Information (A2), dass der gemessene Testwert (A1) von einer Malaria-positiven Blutprobe stammt, und der gemessene Testwert (B1), der für die zuvor festgelegte Bluttestkomponente unter Verwendung einer als Malaria-negativ bekannten Blutprobe (B) als ein Messobjekt erhalten wurde, und die Information (B2), dass der gemessene Testwert (B1) von einer Malaria-negativen Blutprobe stammt.

7. Blutanalysevorrichtung nach Anspruch 6, wobei die wichtige Testkomponente eines oder mehrere aufweist, die ausgewählt sind aus der Gruppe
ob eine Frequenzspitze in einem Volumenbereich von 25 fL bis 45 fL in einer Volumenfrequenzverteilung von Leukozyten in einer zu analysierenden Blutprobe (M1) vorhanden ist,
die Leukozytenanzahl einer zu analysierenden Blutprobe (M1),
die Blutplättchenanzahl einer zu analysierenden Blutprobe (M1)
und der CRP-Wert einer zu analysierenden Blutprobe (M1).

8. Blutanalysevorrichtung nach Anspruch 1, wobei der Bestimmungsteil (430) dazu eingerichtet ist, einen Grad an Relevanz für jede von zuvor festgelegten Bluttestkomponenten für Malaria-Positivität, die durch das folgende zweite erlernte Modell (M2) spezifiziert werden, zu verwenden und auf der Grundlage eines gemessenen Testwertes der zuvor festgelegten Bluttestkomponente und des spezifizierten Grades an Relevanz zu bestimmen, ob die Blutprobe (M1) eine Wahrscheinlichkeit besitzt, Malaria-positiv zu sein,
wobei das zweite erlernte Modell (M2) durch maschinelles Lernen, welches der zuvor festgelegten Bluttestkomponenten in welchen Grad mit Malaria-Positivität in Zusammenhang steht, gebildet wird, indem als Lehrerdaten verwendet werden der gemessene Testwert (A1), der für die zuvor festgelegte Bluttestkomponente unter Verwendung einer als Malaria-positiv bekannten Blutprobe (A) als ein Messobjekt erhalten wurde, und die Information (A2), dass der gemessene Testwert (A1) von einer Malaria-positiven Blutprobe stammt, und der gemessene Testwert (B1), der für die zuvor festgelegte Bluttestkomponente unter Verwendung einer als Malaria-negativ bekannten Blutprobe (B) als ein Messobjekt erhalten wurde, und die Information (B2), dass der gemessene Testwert (B1) von einer Malaria-negativen Blutprobe stammt.

9. Computerprogramm, das es einem Computer erlaubt, einen Schritt des Empfangens gemessener Testwerte einer zu analysierenden Blutprobe (M1) durchzuführen, wobei die gemessenen Testwerte Messwerte jeweiliger zuvor festgelegter Bluttestkomponenten, einschließlich eines CRP-Wertes und eines Zählwertes von Blutzellen, sind, und
einen Schritt des Bestimmens, ob die Blutprobe (M1) eine Wahrscheinlichkeit besitzt, Malaria-positiv zu sein, auf der Grundlage eines gemessenen Testwertes für mindestens einen Zählwert von Blutzellen und eines gemessenen Testwertes bezüglich des CRP-Wertes.

10. Computerprogramm nach Anspruch 9, wobei der Computer einen Schritt des Empfangens eines Volumenmesswertes eines Partikels in der Probenflüssigkeit, die die Blutprobe (M1) umfasst, durchführt,
einen Schritt des Empfangens eines CRP-Parameterwertes in der Probenflüssigkeit,
einen Schritt des Bestimmens, ob ein Frequenzspitzenteil innerhalb eines für das Malariaplasmodium einzigartigen Volumenbereichs in der Volumenfrequenzverteilung auf der Grundlage des Volumenmesswertes vorhanden ist,
einen Schritt des Bestimmens, ob der CRP-Wert auf der Grundlage des CRP-Parameterwertes nicht kleiner als ein im Voraus bestimmter Schwellenwert ist, und
einen Schritt des Bestimmens, dass die Blutprobe (M1) eine hohe Wahrscheinlichkeit besitzt, Malaria-positiv zu sein, wenn der Frequenzspitzenteil vorhanden ist oder wenn der CRP-Wert nicht kleiner als der Schwellenwert ist.

11. Computerprogramm nach Anspruch 10, des Weiteren umfassend ein Computerprogramm, das es einem Computer erlaubt, einen Schritt des Bestimmens durchzuführen, dass die Blutprobe (M1) eine hohe Wahrscheinlichkeit besitzt, Malaria-positiv zu sein, wenn der Frequenzspitzenteil vorhanden ist und der CRP-Wert nicht kleiner als der Schwellenwert ist.

12. Computerprogramm nach Anspruch 9, wobei der Bestimmungsschritt einen Schritt umfasst, eine wichtige Testkomponente bezüglich Malaria-Positivität, die anhand zuvor festgelegter Bluttestkomponenten durch das folgende erste erlernte Modell spezifiziert wird, zu verwenden und auf der Grundlage eines gemessenen Testwertes der wichtigen Testkomponente zu bestimmen, ob die Blutprobe (M1) eine Wahrscheinlichkeit besitzt, Malaria-positiv zu sein,
wobei das erste erlernte Modell durch maschinelles Lernen, welches der zuvor festgelegten Bluttestkomponenten mit Malaria-Positivität in Zusammenhang steht, gebildet wird, indem als Lehrerdaten verwendet werden der gemessene Testwert (A1), der für die zuvor festgelegte Bluttestkomponente unter Verwendung einer als Malaria-positiv bekannten Blutprobe (A) als ein Messobjekt erhalten wurde, und die Information (A2), dass der gemessene Testwert (A1) von einer Malaria-positiven Blutprobe stammt, und der gemessene Testwert (B1), der für die zuvor festgelegte Bluttestkomponente unter Verwendung einer als Malaria-negativ bekannten Blutprobe (B) als ein Messobjekt erhalten wurde, und die Information (B2), dass der gemessene Testwert (B1) von einer Malaria-negativen Blutprobe stammt.

13. Computerprogramm nach Anspruch 9, wobei der Bestimmungsschritt einen Schritt umfasst, einen Grad an Relevanz für jede von zuvor festgelegten Bluttestkomponenten für Malaria-Positivität, die durch das folgende zweite erlernte Modell (M2) spezifiziert werden, zu verwenden und auf der Grundlage eines gemessenen Testwertes der zuvor festgelegten Bluttestkomponente und des spezifizierten Grades an Relevanz zu bestimmen, ob die Blutprobe (M1) eine Wahrscheinlichkeit besitzt, Malaria-positiv zu sein,
wobei das zweite erlernte Modell (M2) durch maschinelles Lernen, welches der zuvor festgelegten Bluttestkomponenten in welchen Grad mit Malaria-Positivität in Zusammenhang steht, gebildet wird, indem als Lehrerdaten verwendet werden der gemessene Testwert (A1), der für die zuvor festgelegte Bluttestkomponente unter Verwendung einer als Malaria-positiv bekannten Blutprobe (A) als ein Messobjekt erhalten wurde, und die Information (A2), dass der gemessene Testwert (A1) von einer Malaria-positiven Blutprobe stammt, und der gemessene Testwert (B1), der für die zuvor festgelegte Bluttestkomponente unter Verwendung einer als Malaria-negativ bekannten Blutprobe (B) als ein Messobjekt erhalten wurde, und die Information (B2), dass der gemessene Testwert (B1) von einer Malaria-negativen Blutprobe stammt.

14. Verfahren zum Analysieren einer Blutprobe, umfassend einen Schritt des Vorbereitens gemessener Testwerte einer zu analysierenden Blutprobe (M1), wobei die gemessenen Testwerte Messwerte jeweiliger zuvor festgelegter Bluttestkomponenten, einschließlich eines CRP-Wertes und eines Zählwertes von Blutzellen, sind, und
einen Schritt des Bestimmens, ob die Blutprobe (M1) eine Wahrscheinlichkeit besitzt, Malaria-positiv zu sein, auf der Grundlage eines gemessenen Testwertes für mindestens einen Zählwert von Blutzellen und eines gemessenen Testwertes bezüglich des CRP-Wertes.

15. Blutprobenanalyseverfahren nach Anspruch 14, wobei der Bestimmungsschritt einen Schritt umfasst, eine wichtige Testkomponente bezüglich Malaria-Positivität, die anhand zuvor festgelegter Bluttestkomponenten durch das folgende erste erlernte Modell spezifiziert wird, zu verwenden und auf der Grundlage eines gemessenen Testwertes der wichtigen Testkomponente zu bestimmen, ob die Blutprobe (M1) eine Wahrscheinlichkeit besitzt, Malaria-positiv zu sein,
wobei das erste erlernte Modell durch maschinelles Lernen, welches der zuvor festgelegten Bluttestkomponenten mit Malaria-Positivität in Zusammenhang steht, gebildet wird, indem als Lehrerdaten verwendet werden: der gemessene Testwert (A1), der für die zuvor festgelegte Bluttestkomponente unter Verwendung einer als Malaria-positiv bekannten Blutprobe (A) als ein Messobjekt erhalten wurde, und die Information (A2), dass der gemessene Testwert (A1) von einer Malaria-positiven Blutprobe stammt, und der gemessene Testwert (B1), der für die zuvor festgelegte Bluttestkomponente unter Verwendung einer als Malaria-negativ bekannten Blutprobe (B) als ein Messobjekt erhalten wurde, und die Information (B2), dass der gemessene Testwert (B1) von einer Malaria-negativen Blutprobe stammt.

16. Blutprobenanalyseverfahren nach Anspruch 14, wobei der Bestimmungsschritt einen Schritt umfasst, einen Grad an Relevanz für jeden von zuvor festgelegten Bluttestkomponenten für Malaria-Positivität, der durch das folgende zweite erlernte Modell (M2) spezifiziert ist, zu verwenden und auf der Grundlage eines gemessenen Testwertes der zuvor festgelegten Bluttestkomponente und des spezifizierten Grades an Relevanz zu bestimmen, ob die Blutprobe (M1) eine Wahrscheinlichkeit besitzt, Malaria-positiv zu sein,
wobei das zweite erlernte Modell (M2) durch maschinelles Lernen, welches der zuvor festgelegten Bluttestkomponenten in welchen Grad mit Malaria-Positivität in Zusammenhang steht, gebildet wird, indem als Lehrerdaten verwendet werden der gemessene Testwert (A1), der für die zuvor festgelegte Bluttestkomponente unter Verwendung einer als Malaria-positiv bekannten Blutprobe (A) als ein Messobjekt erhalten wurde, und die Information (A2), dass der gemessene Testwert (A1) von einer Malaria-positiven Blutprobe stammt, und der gemessene Testwert (B1), der durch Messen für die zuvor festgelegte Bluttestkomponente unter Verwendung einer als Malaria-negativ bekannten Blutprobe (B) als ein Messobjekt erhalten wurde, und die Information (B2), dass der gemessene Testwert (B1) von einer Malaria-negativen Blutprobe stammt.

## Revendications

1. Dispositif d'analyse de sang comprenant une partie réception de valeurs mesurées (410) et une partie détermination (430), dans lequel
la partie réception de valeurs mesurées (410) reçoit des valeurs de test mesurées d'un échantillon de sang (M1) à analyser,
les valeurs de test mesurées sont des valeurs mesurées d'éléments de test sanguin prédéterminés respectifs incluant une valeur de CRP basée sur une valeur de paramètre de CRP et une valeur de comptage de cellules sanguines,
la partie réception de valeurs mesurées (410) possède une partie réception de valeur de CRP pour recevoir une valeur de paramètre de CRP dans un échantillon de liquide comprenant le spécimen sanguin (M1), et
la partie détermination (430) détermine si le spécimen sanguin (M1) a une probabilité d'être positif au paludisme sur la base d'une valeur de test mesurée d'au moins une valeur de comptage de cellules sanguines et d'une valeur de test mesurée spécifique concernant la valeur de CRP.

2. Dispositif d'analyse de sang selon la revendication 1, dans lequel la partie réception de valeurs mesurées (410) possède une partie réception de valeur de volume qui reçoit une valeur mesurée de volume d'une particule dans l'échantillon de liquide comprenant le spécimen sanguin (M1), et
la partie détermination (430) détermine que le spécimen sanguin (M1) a une forte probabilité d'être positif au paludisme lorsqu'une partie pic de fréquence existe dans une plage de volume prédéterminée dans une distribution de fréquence de volume sur la base de la valeur mesurée de volume, ou lorsque la valeur de CRP basée sur la valeur de paramètre de CRP n'est pas inférieure à une valeur seuil déterminée à l'avance.

3. Dispositif d'analyse de sang selon la revendication 2, comprenant en outre une chambre de mesure des leucocytes en tant que première partie de mesure des particules (11), dans lequel
dans la chambre de mesure des leucocytes, un échantillon de sang (M1) à fournir est soumis à un traitement d'hémolyse pour lyser les érythrocytes et dilué pour former l'échantillon de liquide, et un volume de chacun des leucocytes et de particules autres que les leucocytes dans l'échantillon de liquide est mesuré, et
la partie réception de valeur de volume reçoit la valeur mesurée de volume à partir de la première partie de mesure des particules (11).

4. Dispositif d'analyse de sang selon la revendication 2 ou 3, comprenant en outre une partie mesure de CRP (13) qui mesure la valeur de paramètre de CRP, dans lequel
la partie réception de valeur de CRP reçoit la valeur de paramètre de CRP à partir de la partie mesure de CRP (13).

5. Dispositif d'analyse de sang selon l'une quelconque des revendications 2 à 4, dans lequel la partie détermination (430) détermine que le spécimen sanguin (M1) a une forte probabilité d'être positif au paludisme lorsque la partie pic de fréquence existe dans la plage de volume prédéterminée dans la distribution de fréquence de volume sur la base de la valeur mesurée de volume, et lorsque la valeur de CRP basée sur la valeur de paramètre de CRP n'est pas inférieure à la valeur de seuil déterminée à l'avance.

6. Dispositif d'analyse de sang selon la revendication 1, dans lequel la partie détermination (430) est configurée pour utiliser un élément de test important lié à la positivité au paludisme qui est spécifié à partir d'éléments de test sanguin prédéterminés par le premier modèle d'apprentissage suivant, et déterminer si le spécimen sanguin (M1) a une probabilité d'être positif au paludisme sur la base d'une valeur de test mesurée de l'élément de test important,
dans lequel le premier modèle d'apprentissage est formé par apprentissage automatique des éléments de test sanguin prédéterminés qui sont liés à la positivité au paludisme en utilisant, comme données d'apprentissage, la valeur d'analyse mesurée (A1) obtenue pour l'élément de test sanguin prédéterminé en utilisant le spécimen sanguin (A) connu pour être positif au paludisme comme objet de mesure, et l'information (A2) selon laquelle la valeur d'analyse mesurée (A1) est celle d'un échantillon de sang positif au paludisme, et la valeur d'analyse mesurée (B1) obtenue pour l'élément de test sanguin prédéterminé en utilisant le spécimen sanguin (B) connu pour être négatif au paludisme comme objet de mesure, et l'information (B2) selon laquelle la valeur d'analyse mesurée (B1) est celle d'un échantillon de sang négatif au paludisme.

7. Dispositif d'analyse de sang selon la revendication 6, dans lequel l'élément de test important comporte un ou plusieurs éléments choisis dans le groupe constitué par
le fait de savoir si un pic de fréquence existe dans une plage de volume de 25 fL à 45 fL dans une distribution de fréquence de volume des leucocytes dans un échantillon de sang (M1) à analyser,
la numération leucocytaire d'un échantillon de sang (M1) à analyser,
la numération plaquettaire d'un échantillon de sang (M1) à analyser, et
la valeur de CRP d'un échantillon de sang (M1) à analyser.

8. Dispositif d'analyse de sang selon la revendication 1, dans lequel la partie détermination (430) est configurée pour utiliser un degré de pertinence pour chacun d'éléments de test sanguin prédéterminés pour la positivité au paludisme qui est spécifié par le second modèle d'apprentissage suivant (M2), et déterminer si le spécimen sanguin (M1) a une probabilité d'être positif au paludisme sur la base d'une valeur de test mesurée de l'élément de test sanguin prédéterminé et du degré de pertinence spécifié,
dans lequel le second modèle d'apprentissage (M2) est formé par apprentissage automatique des éléments de test sanguin prédéterminés qui sont liés à la positivité au paludisme, et dans quelle mesure, en utilisant, comme données d'apprentissage, la valeur de test mesurée (A1) obtenue pour l'élément de test sanguin prédéterminé en utilisant le spécimen sanguin (A) connu pour être positif au paludisme comme objet de mesure, et l'information (A2) selon laquelle la valeur d'analyse mesurée (A1) est celle d'un échantillon de sang positif au paludisme, et la valeur d'analyse mesurée (B1) obtenue pour l'élément de test sanguin prédéterminé en utilisant le spécimen sanguin (B) connu pour être négatif au paludisme comme objet de mesure, et l'information (B2) selon laquelle la valeur d'analyse mesurée (B1) est celle d'un échantillon de sang négatif au paludisme.

9. Programme informatique qui permet à un ordinateur d'effectuer une étape consistant à recevoir des valeurs de test mesurées d'un échantillon de sang (M1) à analyser, dans lequel les valeurs de test mesurées sont des valeurs mesurées d'éléments de test sanguin prédéterminés respectifs incluant une valeur de CRP et une valeur de comptage de cellules sanguines, et
une étape consistant à déterminer si le spécimen sanguin (M1) a une probabilité d'être positif au paludisme sur la base d'une valeur de test mesurée pour au moins une valeur de comptage de cellules sanguines et d'une valeur de test mesurée concernant la valeur de CRP.

10. Programme informatique selon la revendication 9, dans lequel l'ordinateur effectue une étape consistant à recevoir une valeur mesurée de volume d'une particule dans l'échantillon de liquide comprenant le spécimen sanguin (M1),
une étape consistant à recevoir une valeur de paramètre de CRP dans l'échantillon de liquide,
une étape consistant à déterminer si une partie pic de fréquence existe dans une plage de volume propre au plasmodium du paludisme dans la distribution de fréquence de volume sur la base de la valeur mesurée de volume,
une étape consistant à déterminer si la valeur de CRP basée sur la valeur de paramètre de CRP n'est pas inférieure à une valeur seuil déterminée à l'avance, et
une étape consistant à déterminer que le spécimen sanguin (M1) a une forte probabilité d'être positif au paludisme lorsque la partie pic de fréquence existe ou lorsque la valeur de CRP n'est pas inférieure à la valeur seuil.

11. Programme informatique selon la revendication 10, comprenant en outre un programme informatique qui permet à un ordinateur d'effectuer une étape consistant à déterminer que le spécimen sanguin (M1) a une forte probabilité d'être positif au paludisme lorsque la partie pic de fréquence existe et que la valeur de CRP n'est pas inférieure à la valeur seuil.

12. Programme informatique selon la revendication 9, dans lequel l'étape de détermination comprend une étape consistant à utiliser un élément de test important lié à la positivité au paludisme qui est spécifié à partir d'éléments de test sanguin prédéterminés par le premier modèle d'apprentissage suivant, et à déterminer si le spécimen sanguin (M1) a une probabilité d'être positif au paludisme sur la base d'une valeur de test mesurée de l'élément de test important,
dans lequel le premier modèle d'apprentissage est formé par apprentissage automatique des éléments de test sanguin prédéterminés qui sont lié à la positivité au paludisme en utilisant, comme données d'apprentissage, la valeur d'analyse mesurée (A1) obtenue pour l'élément de test sanguin prédéterminé en utilisant le spécimen sanguin (A) connu pour être positif au paludisme comme objet de mesure, et l'information (A2) selon laquelle la valeur d'analyse mesurée (A1) est celle d'un échantillon de sang positif au paludisme, et la valeur d'analyse mesurée (B1) obtenue pour l'élément de test sanguin prédéterminé en utilisant le spécimen sanguin (B) connu pour être négatif au paludisme comme objet de mesure, et l'information (B2) selon laquelle la valeur d'analyse mesurée (B1) est celle d'un échantillon de sang négatif au paludisme.

13. Programme informatique selon la revendication 9, dans lequel l'étape de détermination comprend une étape consistant à utiliser un degré de pertinence pour chacun des éléments de test sanguin prédéterminés pour la positivité au paludisme qui est spécifié par le second modèle d'apprentissage suivant (M2), et à déterminer si le spécimen sanguin (M1) a une probabilité d'être positif au paludisme sur la base d'une valeur de test mesurée de l'élément de test sanguin prédéterminé et du degré de pertinence spécifié,
dans lequel le second modèle d'apprentissage (M2) est formé par apprentissage automatique des éléments de test sanguin prédéterminés qui sont liés à la positivité au paludisme, et dans quelle mesure, en utilisant, comme données d'apprentissage, la valeur de test mesurée (A1) obtenue pour l'élément de test sanguin prédéterminé en utilisant le spécimen sanguin (A) connu pour être positif au paludisme comme objet de mesure, et l'information (A2) selon laquelle la valeur d'analyse mesurée (A1) est celle d'un échantillon de sang positif au paludisme, et la valeur d'analyse mesurée (B1) obtenue pour l'élément de test sanguin prédéterminé en utilisant le spécimen sanguin (B) connu pour être négatif au paludisme comme objet de mesure, et l'information (B2) selon laquelle la valeur d'analyse mesurée (B1) est celle d'un échantillon de sang négatif au paludisme.

14. Procédé d'analyse d'un échantillon de sang, comprenant une étape consistant à préparer des valeurs de test mesurées d'un échantillon de sang (M1) à analyser, dans lequel les valeurs de test mesurées sont des valeurs mesurées d'éléments de test sanguin prédéterminés respectifs comportant une valeur de CRP et une valeur de comptage de cellules sanguines, et
une étape consistant à déterminer si le spécimen sanguin (M1) a une probabilité d'être positif au paludisme sur la base d'une valeur de test mesurée pour au moins une valeur de comptage de cellules sanguines et d'une valeur de test mesurée concernant la valeur de CRP.

15. Procédé d'analyse d'un échantillon de sang selon la revendication 14, dans lequel l'étape de détermination comprend une étape consistant à utiliser un élément de test important lié à la positivité au paludisme qui est spécifié parmi des éléments de test sanguin prédéterminés par le premier modèle d'apprentissage suivant, et à déterminer si le spécimen sanguin (M1) a une probabilité d'être positif au paludisme sur la base d'une valeur de test mesurée de l'élément de test important,
dans lequel le premier modèle d'apprentissage est formé par apprentissage automatique des éléments de test sanguin prédéterminés qui sont liés à la positivité au paludisme en utilisant, comme données d'apprentissage, la valeur d'analyse mesurée (A1) obtenue pour l'élément de test sanguin prédéterminé en utilisant le spécimen sanguin (A) connu pour être positif au paludisme comme objet de mesure, et l'information (A2) selon laquelle la valeur d'analyse mesurée (A1) est celle d'un échantillon de sang positif au paludisme, et la valeur d'analyse mesurée (B1) obtenue pour l'élément de test sanguin prédéterminé en utilisant le spécimen sanguin (B) connu pour être négatif au paludisme comme objet de mesure, et l'information (B2) selon laquelle la valeur d'analyse mesurée (B1) est celle d'un échantillon de sang négatif au paludisme.

16. Procédé d'analyse d'un échantillon de sang selon la revendication 14, dans lequel l'étape de détermination comprend une étape consistant à utiliser un degré de pertinence pour chacun d'éléments de test sanguin prédéterminés pour la positivité au paludisme qui est spécifié par le second modèle d'apprentissage suivant (M2), et à déterminer si le spécimen sanguin (M1) a une probabilité d'être positif au paludisme sur la base d'une valeur de test mesurée de l'élément de test sanguin prédéterminé et du degré de pertinence spécifié,
dans lequel le second modèle d'apprentissage (M2) est formé par apprentissage automatique des éléments de test sanguin prédéterminés qui sont liés à la positivité au paludisme, et dans quelle mesure, en utilisant, comme données d'apprentissage, la valeur de test mesurée (A1) obtenue pour l'élément de test sanguin prédéterminé en utilisant le spécimen sanguin (A) connu pour être positif au paludisme comme objet de mesure, et l'information (A2) selon laquelle la valeur d'analyse mesurée (A1) est celle d'un échantillon de sang positif au paludisme, et la valeur d'analyse mesurée (B1) obtenue par une mesure pour l'élément de test sanguin prédéterminé en utilisant un spécimen sanguin (B) connu pour être négatif au paludisme comme objet de mesure, et l'information (B2) selon laquelle la valeur d'analyse mesurée (B1) est celle d'un échantillon de sang négatif au paludisme.
